# EUROPEAN PATENT APPLICATION

(11) **EP 1 857 116 A1**
(43) Date of publication of application: **21.11.2007**
(21) Application number: 06114796.3
(22) Date of filing: 31.05.2006
(51) Int. Cl.: A61K 39/215, C07K 16/10

(54) **Antigen binding polypeptides against spike glycoprotein (S2) of bovine coronavirus**

(30) Priority: 19.05.2006 EP 06114237
(71) Applicant: Novoplant GmbH, 06466 Gatersleben (DE)
(72) Inventor: Zimmerman, Jana, Dr., 06766 Bobbau (DE); Riehl, Marcus, Dr., 34628 Willingshausen (DE); Giersberg, Martin, Dr., 06484 Quedlinburg (DE)
(74) Representative: Neuefeind, Regina

(57) **Abstract**

The present invention relates to antigen-binding polypeptides, particularly antibody fragments, and especially single-chain antibodies, which specifically bind to bovine coronavirus spike glycoprotein (S2). The polypeptides neutralize the cytopathic changes of cells by bovine coronavirus and/or have a protective and/or curative effect on infection of mammals, especially calves and cattle, by viruses expressing spike glycoprotein (S2). The polypeptides may be expressed in a prokaryotic or eukaryotic host cell, preferably a bacterial, yeast or plant cell. Also provided are transgenic plants or parts thereof and microorganisms expressing the polynucleotides of the present invention. The plants or plant parts expressing the polypeptides may be used as fodder for mammals and especially calves and cattle. Furthermore, the invention relates to polynucleotides encoding said spike glycoprotein (S2)-binding polypeptides, vectors comprising these polynucleotides, host cells comprising these polynucleotides or vectors, compositions comprising said spike glycoprotein (S2)-binding polypeptides, and methods of producing said polypeptides.

## Description

### BACKGROUND OF THE INVENTION

Bovine coronavirus (BCV) is a member of the Coronaviridae, causing severe diarrhea in calves and adult cattle. In adult animals, BCV infection is usually subclinical. However, the virus has also been identified as the cause of winter dysentery in adult cows (Mebus C.A. Am. J. Vet. Res. 1973 Feb;34(2):145-50; Saif L.J. Vet. Rec. 1988 Sep 10;123(11):300-1). Winter dysentery is characterized by a sudden onset of diarrhea that rapidly affects many adult cattle resulting in a dramatic reduction of milk production (Traven M. Vet. Microbiol. 2001 Jul 26;81(2):127-51).

The virion contains five major structural proteins: the nucleocapsid protein (N), the transmembrane protein (M), the the hemagglutinin/esterase protein (HE), the small membrane protein (E) and the spike protein (S) (Cavanagh D. Virology 1990 May;176(1):306-7; Lai M.M. and Cavanagh D. Adv. Virus Res. 1997;48:1-100). The S glycoprotein forms the typical coronavius morphology. Its precursor protein is cleaved into a N-terminal half (S1) and a C-terminal half (S2) both with an approximate molecular weight of 100kDa (Spaan W. J. Gen. Virol. 1988 Dec; 69 (Pt 12):2939-52; St Cyr-Coats K.S. Arch. Virol. 1988; 103(1-2):35-45).

The S glycoprotein is involved in virus attachment to permissive cells, virus-induced cell fusion, elicitation of neutralizing antibodies and cell-mediated immunity (Sturman L.S. J. Virol. 1985 Dec;56(3):904-11)*. Anti-S monoclonal antibodies (Mabs) recognize conformational epitopes in two distinct antigenic sites, A and B (*Deregt D. and Babiuk L.A. Virology 1987 Dec;161(2):410-20*)*.

The S1 subunit presumably forms the bulbous upper part of the projections and bears type-specific neutralization determinants. The S2 subunit has been predicted to contain two long α helices which are involved in the formation of the stalk of the peplomer *and elicits the production of group-specific monoclonal antibodies (*Vautherot J.F. J. Gen. Virol. 1992 Dec; 73 (Pt 12):3289-94*).*

Antibodies are proteins which are produced and secreted by B lymphocytes / plasma cells and which reversibly and non-covalently bind to their specific antigen. IgG antibodies consist of four polypeptide chains, two identical heavy (H) chains of about 50-70 kD and two identical light (L) chains of about 25 kD. Each chain consists of a constant (C) and a variable (V) region. The constant region is composed of three to four constant domains in case of the heavy chain (CH₁ - CH₄) and one constant domain in case of the light chain (CL).

The variable region of the heavy chain (VH) and of the light chain (VL) are each composed of one domain, consisting of about 110 amino acids. The VH and the VL region each comprise three complementarity determining regions (CDR1, CDR2, CDR3) which represent the most variable regions of the antibody and confer antigen binding specificity. The framework regions in between the CDRs are relatively conserved.

It has been shown that fragments of a whole antibody containing the CDR regions can still perform the binding function. These smaller molecules can be produced effectively e.g. in *E. coli,* yeasts and plant cells whereas whole IgG molecules still need to be produced in mammalian cell culture. Furthermore unwanted effects triggered by the constant part of the molecule are avoided, such as complement activation. However, the clearance rate is higher for antibody fragments, resulting in a shorter half-life upon systemic application.

An antibody "Fab" fragment (fragment antigen binding) is composed of a complete light chain linked to the N-terminal part of a heavy chain, consisting of the variable region (VH) and one constant domain (CH₁). The Fab fragment may be generated e.g. by papain hydrolysis of an antibody.

Gene technology methods such as phage display allow the production of a recombinant single-chain antibody "Fv" fragment (fragment variable) which is a truncated Fab fragment and consists of the variable region of a heavy chain and the variable region of a light chain which are linked to one another by an artificial peptide (scFv, single-chain fragment variable). "Phage display" is a standard technique for the presentation of distinct peptides, polypeptides or proteins on the surface of bacteriophages. It allows the identification of the exposed peptides, polypeptides or proteins via their binding properties. The technique is used for the establishment of libraries of peptide, polypeptide or protein variants such as different antibody fragments. For this, mammals such as mice are immunized against the antigen of interest and lymphocytes are isolated, e.g. from the spleen. Then, mRNA is isolated therefrom, and a cDNA library is generated by cloning the cDNA into a phage display vector.

The phage display library generated in this way contains recombinant antibody fragments. These can be analyzed for their antigen binding specificities e.g. by the panning procedure. The random library is screened for protein-protein interactions. Interacting clones of the library are enriched, and the plasmid DNA from individual clones can be isolated and sequenced.

It is an object of the present invention to provide novel antigen-binding polypeptides, particularly antibody fragments, and especially single-chain antibodies, which specifically bind to bovine coronavirus spike glycoprotein (S2). A further object of the present invention is to provide polynucleotides and/or vectors encoding these polypeptides, prokaryotic or eukaryotic host cells comprising these polynucleotides and/or vectors, transgenic plants or parts thereof or microorganisms containing the polynucleotides and/or vectors of the present invention, as well as compositions comprising said spike glycoprotein (S2)-binding polypeptides. Furthermore, the present invention also provides a method by which said spike glycoprotein (S2)-binding polypeptides may be produced.

These and further objects are achieved by the subject-matter of the patent claims, which will become clear from the following description.

### DETAILED DESCRIPTION OF THE INVENTION

In a first aspect the present invention relates to an antigen-binding polypeptide which specifically binds to bovine coronavirus spike glycoprotein (S2). The term "antigen-binding polypeptide" as used herein means a polypeptide or protein which, according to the invention, specifically binds to bovine coronavirus spike glycoprotein (S2). The term "specific binding" as used herein means that the polypeptide of the present invention does not show any significant binding to molecules other than to bovine coronavirus spike glycoprotein (S2) or fragments thereof. Usually this binding is reversible and/or non-covalent.

Other preferred embodiments of the present invention are defined in the respective subclaims.

In one preferred embodiment, the antigen-binding polypeptide of the present invention binds to a polypeptide comprising the amino acid sequence of bovine coronavirus spike glycoprotein (S2) (SEQ ID NO: 1) and/or fragments thereof.

In another aspect the present invention relates to an antigen-binding polypeptide comprising a first polypeptide comprising the antigen-binding portion of the variable region of an antibody heavy chain, and a second polypeptide comprising the antigen-binding portion of the variable region of an antibody light chain, wherein said antigen-binding polypeptide specifically binds to bovine coronavirus spike glycoprotein (S2).

The term "heavy chain" or "light chain" as used herein means the conventional antibody polypeptide heavy chain (H) or light chain (L), respectively. The term "variable region" as used herein means the conventional antibody variable region or variable domain or variable fragment (V or Fv) which confers antigen binding specificity. Variable regions employed in the invention may be derived from any germline or rearranged variable domain, or may be a synthetic variable domain based on consensus sequences of known variable domains. Usually the variable region of the heavy chain (VH) and the variable region of the light chain (VL) each comprise three complementarity determining regions CDR1, CDR2 and CDR3.

Seven antigen-binding polypeptides which specifically bind to bovine coronavirus spike glycoprotein (S2) have been generated via immunization of mice, cloned into a phage display library, tested for their spike glycoprotein (S2)-binding specificity, isolated and sequenced. They carry the designations DA6, DA10, DA11, DA25, DA30, DA31 and DA43. These polypeptides are single-chain polypeptides. Their full length amino acid sequences are depicted in SEQ ID NOs: 2, 3, 4, 5, 6, 7 and 8, and their full length nucleotide sequences are depicted in SED ID NOs: 9, 10, 11, 12, 13, 14 and 15.

Surprisingly it was found that these polypeptides, all comprising an antigen-binding portion of a VH chain and an antigen-binding portion of a VL chain, are characterized by similar CDR1, CDR2 and CDR3 regions (tables 1 and 2). Therefore it was possible to characterize these spike glycoprotein (S2)-binding single-chain polypeptides by means of amino acid and nucleotide consensus sequences of their CDRs. The locations of the CDRs were determined by reference to Kabat E.A. et al. (Sequences of Proteins of Immunological Interest, 4th Edition, U.S. Department of Health and Human Services, 1987) and updates thereof, now available on the Internet.

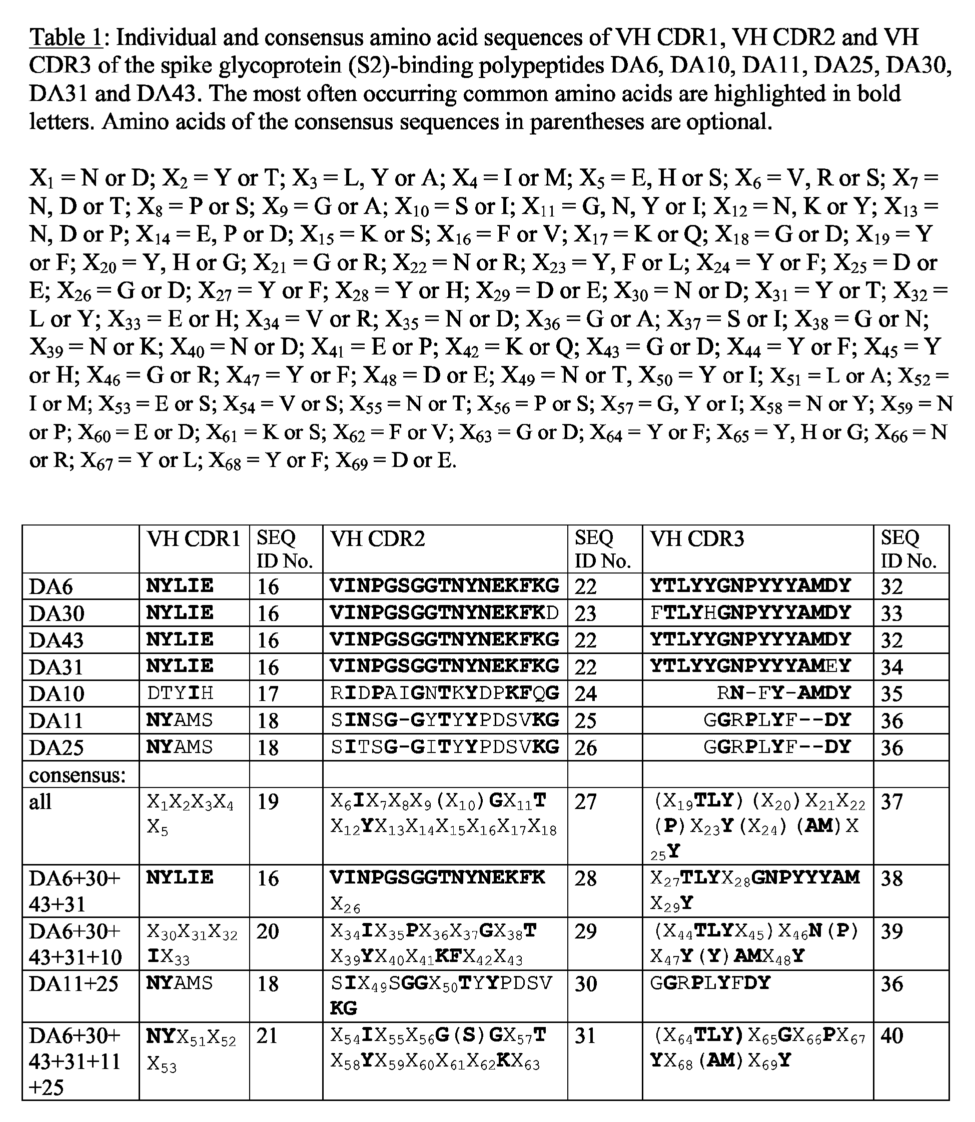

The amino acid sequences which are designated in claims 5 to 10 refer to the SEQ ID NOs of table 1. For the purpose of clarification of the sequence protocol, the consensus sequences of table 1 which contain amino acids in parentheses (SEQ ID NOs: 27, 31, 37, 39 and 40) are defined in the sequence protocol as follows:
SEQ ID NO: 27 X₆IX₇X₈X₉X₁₀GX₁₁TX₁₂YX₁₃X₁₄X₁₅X₁₆X₁₇X₁₈
SEQ ID NO: 138 X₆IX₇X₈X₉GX₁₁TX₁₂YX₁₃X₁₄X₁₅X₁₆X₁₇X₁₈

SEQ ID NO: 31 X₅₄IX₅₅X₅₆GSGX₅₇TX₅₈YX₅₉X₆₀X₆₁X₆₂KX₆₃
SEQ ID NO: 139 X₅₄IX₅₅X₅₆GGX₅₇TX₅₈YX₅₉X₆₀X₆₁X₆₂KX₆₃

SEQ ID NO: 37 X₁₉TLYX₂₀X₂₁X₂₂PX₂₃YX₂₄AMX₂₅Y
SEQ ID NO: 140 X₂₁X₂₂X₂₃YAMX₂₅Y
SEQ ID NO: 141 X₂₀X₂₁X₂₂PX₂₃YX₂₄X₂₅Y

SEQ ID NO: 39 X₄₄TLYX₄₅X₄₆NPX₄₇YYAMX₄₈Y
SEQ ID NO: 142 X₄₆NX₄₇YAMX₄₈Y

SEQ ID NO: 40 X₆₄TLYX₆₅GX₆₆PX₆₇YX₆₈AMX₆₉Y
SEQ ID NO: 143 X₆₅GX₆₆PX₆₇YX₆₈X₆₉Y

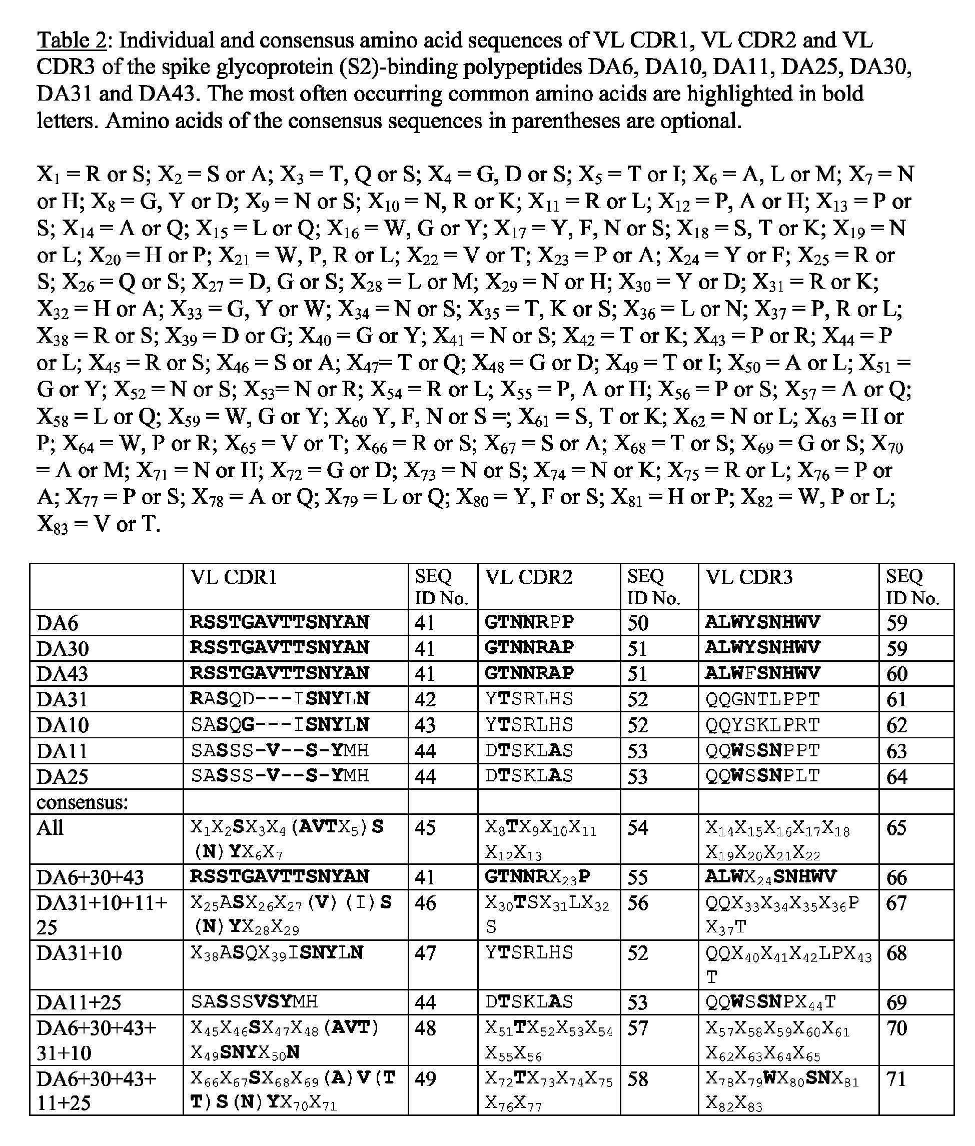

The amino acid sequences which are designated in claims 11 to 16 refer to the SEQ ID NOs of table 2. For the purpose of clarification of the sequence protocol, the consensus sequences of table 2 which contain amino acids in parentheses (SEQ ID NOs: 45, 46, 48, 49,) are defined in the sequence protocol as follows:
SEQ ID NO: 45 X₁X₂SX₃X₄AVTX₅SNYX₆X₇
SEQ ID NO: 144 X₁X₂SX₃X₄X₅SNYX₆X₇
SEQ ID NO: 145 X₁X₂SX₃X₄VSYX₆X₇

SEQ ID NO: 46 X₂₅ASX₂₆X₂₇ISNYX₂₈X₂₉
SEQ ID NO: 146 X₂₅ASX₂₆X₂₇VSYX₂₈X₂₉

SEQ ID NO: 48 X₄₅X₄₆SX₄₇X₄₈AVTX₄₉SNYX₅₀N
SEQ ID NO: 147 X₄₅X₄₆SX₄₇X₄₈X₄₉SNYX₅₀N

SEQ ID NO: 49 X₆₆X₆₇SX₆₈X₆₉AVTTSNYX₇₀X₇₁
SEQ ID NO: 148 X₆₆X₆₇SX₆₈X₆₉VSNYX₇₀X₇₁

Thus, in a preferred embodiment, the antigen-binding polypeptide of the present invention comprises the antigen-binding portion of an antibody heavy chain variable region, which comprises a VH CDR 1, which has the consensus amino acid sequence depicted in SEQ ID NO: 19, more preferably the sequences depicted in SEQ ID NOs: 16, 17 or 18. Other preferred consensus amino acid sequences are depicted in SEQ ID NOs: 20 and 21. Most preferred for VH CDR1 are the individual amino acid sequences depicted in SEQ ID NOs: 16, 17 or 18.

In another preferred embodiment, the antigen-binding polypeptide of the present invention comprises the antigen-binding portion of an antibody heavy chain variable region, which comprises a VH CDR 2, which has the consensus amino acid sequence depicted in SEQ ID NO: 27, more preferably the sequences depicted in SEQ ID NOs: 24, 28 and 30. Other preferred consensus amino acid sequences are depicted in SEQ ID NO: 29 and 31. Most preferred for VH CDR2 are the individual amino acid sequences depicted in SEQ ID NOs: 22, 23, 24, 25 and 26.

In another preferred embodiment, the antigen-binding polypeptide of the present invention comprises the antigen-binding portion of an antibody heavy chain variable region, which comprises a VH CDR 3, which has the consensus amino acid sequence depicted in SEQ ID NO: 37, more preferably the sequences depicted in SEQ ID NOs: 35, 36 and 38. Other preferred consensus amino acid sequences are depicted in SEQ ID NOs: 39 and 40. Most preferred for VH CDR3 are the individual amino acid sequences depicted in SEQ ID NOs: 32, 33, 34, 35 and 34.

In another preferred embodiment, the antigen-binding polypeptide of the present invention comprises the antigen-binding portion of an antibody light chain variable region, which comprises a VL CDR 1, which has the consensus amino acid sequence depicted in SEQ ID NO: 45, more preferably the sequences depicted in SEQ ID NO: 41, 44 and 47. Other preferred consensus amino acid sequences are depicted in SEQ ID NOs: 46, 48 and 49. Most preferred for VL CDR1 are the individual amino acid sequences depicted in SEQ ID NOs: 41, 42, 43 and 44.

In another preferred embodiment, the antigen-binding polypeptide of the present invention comprises the antigen-binding portion of an antibody light chain variable region, which comprises a VL CDR 2, which has the consensus amino acid sequence depicted in SEQ ID NO: 54, more preferably the sequences depicted in SEQ ID NOs: 52, 53 and 55. Other preferred consensus amino acid sequences are depicted in SEQ ID NOs: 56, 57 and 58. Most preferred for VL CDR2 are the individual amino acid sequences depicted in SEQ ID NOs: 50, 51, 52 and 53.

In another preferred embodiment, the antigen-binding polypeptide of the present invention comprises the antigen-binding portion of an antibody light chain variable region, which comprises a VL CDR 3, which has the consensus amino acid sequence depicted in SEQ ID NO: 65, more preferably the sequences depicted in SEQ ID NOs: 66, 68 and 69. Other preferred consensus amino acid sequences are depicted in SEQ ID NOs: 67, 70 and 71. Most preferred for VL CDR3 are the individual amino acid sequences depicted in SEQ ID NOs: 59, 60, 61, 62, 63 and 64.

According to the invention, these six different CDRs may be combined with each other in any conceivable way. The CDRs may further be carried by any antibody framework regions. Preferably these framework sequences derive from the mouse, as for example in SEQ ID NOs: 2, 3, 4, 5, 6, 7 and 8.

In another preferred embodiment of the invention, the antigen-binding polynucleotide, which specifically binds to bovine coronavirus spike glycoprotein (S2), comprises a first polypeptide comprising the antigen-binding portion of the variable region of an antibody heavy chain, a second polypeptide comprising the antigen-binding portion of the variable region of an antibody light chain, and a peptide linker linking said first and said second polypeptide into a single-chain polypeptide.

The term "single-chain polypeptide" or "single-chain antibody" or "single-chain fragment" as used herein means any polypeptide comprising the variable region Fv of a heavy chain, a linking peptide and the variable region Fv of a light chain, resulting in a single chain Fv or "scFv".

In one preferred embodiment, the single-chain antigen-binding polypeptide according to the invention comprises the amino acid sequence of SEQ ID NOs: 2, 3, 4, 5, 6, 7 and 8.

The sequences in SEQ ID NOs: 2, 3, 4, 5, 6, 7 and 8 may further be altered by replacing the linker sequence (here: K L E E G E F S E A R V, SEQ ID NO: 133) with any other desired linker sequence. Further, the respective amino acid sequences of the heavy chain and of the light chain of SEQ ID NOs: 2, 3, 4, 5, 6, 7 and 8 may be combined with each other to form a single-chain antigen-binding polypeptide.

The present invention also refers to multivalent single-chain antigen-binding polypeptides, comprising two or more single-chain polypeptides according to the invention, which specifically bind to bovine coronavirus spike glycoprotein (S2), and wherein the two or more single-chain polypeptides are linked by a peptide linker. When two single-chain polypeptides are linked by a peptide linker, the polypeptide is called "bivalent".

The peptide linkers linking the polypeptides within a single-chain antigen-binding polypeptide or linking two or more single-chain polypeptides to a multivalent single-chain antigen-binding polypeptide are described in the following.

The peptide linker may be any peptide of any length, preferably in the range of about 1 to 100 amino acids, more preferably in the range of about 12 to 25 amino acids. Preferred linkers of the present invention are e.g. the "yol linker" and the "GS linker". Preferably the peptide linker of the present invention is not susceptible to protease cleavage, particularly to proteases occurring in the gastrointestinal tract of mammals, especially of cattle.

The yol linker used in the present invention has the amino acid sequence K L E E G E F S E A R V (SEQ ID NO: 133). This linker may also be truncated or elongated at the N terminus and/or at the C terminus by one or more amino acids, as described in the literature. For example, in Schmiedl A. et al. (Protein Eng. 2000 Oct;13(10):725-34) the yol linker is shortened by one amino acid at each terminus compared to the above described yol linker.

In order to improve the stability of the yol linker and to delete the two putative trypsin cleavage sites (lysine and arginine), the yol linker may be modified by exchanging lysine (K) against isoleucine (I), arginine (R) against glycine (G) and valine (V) against cysteine (C). The resulting linker carries the designation "my" (modified yol) and has the amino acid sequence I L E E G E F S E A G C (SEQ ID NO: 134). Any peptide linker carrying the consensus amino acid sequence X₁ L E E G E F S E A X₂ X₃ (SEQ ID NO: 135), wherein X₁ = K or I, X₂ = R or G, and X₃ = V or C, is also preferred in the present invention.

The linker may also be a GS-linker. The GS-linker may have the amino acid sequence (GGGGS)₂ (SEQ ID NO: 136), it may also have the amino acid sequence (GGGGS)₃, as described in the literature (Huston J.S. Methods Enzymol. 1991;203:46-88). The GS-linker is a preferred linker for the linking of one or more single-chain antigen-binding polypeptides to a multivalent single-chain polypeptide of the present invention.

In one embodiment of the present invention the antigen-binding polypeptide which specifically binds to bovine coronavirus spike glycoprotein (S2) is a Fab antibody fragment. The term "Fab" (fragment antigen binding) or "Fab antibody" or "Fab fragment" as used herein means a fragment comprising a complete antibody light chain linked to the N-terminal part of a heavy chain, comprising the variable region (VH) and one constant domain (CH1). Fab fragments are commercially available, e.g. generated by the company Morphosys, Martinsried/Planegg, Germany.

Other possible antigen-binding polypeptides of the present invention which specifically bind to bovine coronavirus spike glycoprotein (S2) are F(ab')a fragments (bivalent fragments comprising two linked Fab fragments; Holliger P. and Winter G. Curr. Opin. Biotechnol. 1993 Aug;4(4):446-9) and diabodies (multivalent or multispecific fragments constructed by gene fusion; WO94/13804). The generation of Fab and F(ab')₂ fragments is also described in Liddell, J.E. and I. Weeks (1995) Antibody Technology: A Comprehensive Overview, Bios Scientific: Oxford.

Also preferred in the present invention are non-immunoglobulin polypeptides which specifically bind to bovine coronavirus spike glycoprotein (S2). These may e.g. be ANTICALINS® which are produced by Pieris Proteolab AG (Freising, Germany) or Affilin™ molecules which are produced by Scil Proteins GmbH (Halle, Germany).

ANTICALINS® are derived from lipocalins. Lipocalins are composed of a single polypeptide chain with 160 to 180 amino acid residues. They share a structurally conserved β-barrel supporting four loops at one end, which form the entrance to a binding pocket. The loops exhibit conformational differences between individual lipocalins and give rise to the variety of ligand specificities.

Affilin™ molecules are small non-immunoglobulin proteins based on human cytosolic proteins. Affilin ™ molecules can be selected from two libraries, each of which is based on a different human derived scaffold protein, one of which is gamma crystalline, a human structural eye lens protein. Both human scaffolds are very small, show high temperature stability and are almost resistant to pH changes and denaturing agents. This high stability is mainly due to the expanded beta sheet structure of the proteins.

The antigen-binding polypeptide of the present invention, particularly the antibody fragment and especially the single-chain antibody, which specifically binds to bovine coronavirus spike glycoprotein (S2), preferably neutralizes the infectivity of the coronavirus.

One example of an *in vitro* assay which assesses the neutralizing effect of the antigen-binding polypeptides of the present invention is the "virus neutralization test". Vero cells (African Green Monkey) are grown in MEM medium at 37°C with 5% CO₂. Under standard conditions, the virus induces cytopathic changes of the monolayer. Antigen-binding polypeptides which specifically bind to bovine coronavirus spike glycoprotein (S2) are added to this system to measure their neutralizing effect on cytopathic changes. Specific antigen-binding polypeptides protect the monolayer cells from cytopathic changes caused by the virus.

The term "cytopathic changes" as used herein means especially a rounding up and detachment of the cells (the monolayer) from the bottom of the dish or plate. The neutralization of the cytopathic changes means that these effects are diminished or even prevented. There are many graduations between a normal cell growth in form of a monolayer and a complete cytopathic effect. The changes can be observed by a microscope. For quantification purposes, cytotoxicity assays or cell proliferation assays can be used, for example those from Roche (Cytotoxicity Detection Kit, LDH or Cell Proliferation Reagent, WST-1).

In another preferred embodiment, the antigen-binding polypeptide of the present invention has a protective and/or a curative effect on infection of mammals, preferably cattle, with bovine coronavirus.

In order to assess the protective and/or a curative effect, mammals, especially cattle, can be infected with a Coronavirus strain. The treatment group receives the antigen binding polypeptide of the present invention with the feed. The first dose may be applied before or after infection. Thereafter, the animals receive daily doses of the polypeptide, distributed over one or more daily feed applications, for several days. Animals in the control group are infected without polypeptide administration. Monitoring of the clinical symptoms, microbial examination of the feces (which is of high importance for the spread of an infection in a population) and pathological, microbiological and immunochemical analyses (monitoring the presence of the polypeptides in selected organs) give information about the preventative and curative effect of the orally applied antigen-binding polypeptide.

Therefore, another aspect of the present invention is the use of the antigen-binding polypeptide of the present invention, particularly the antibody fragment and especially the single-chain antibody, which specifically binds to spike glycoprotein (S2), for the manufacture of a medicament for the treatment and/or prevention of infections with bacteria expressing spike glycoprotein (S2). The medicament may be applied to mammals, especially to calves. The administration preferably occurs orally.

The present invention further relates to the use of the spike glycoprotein (S2)-binding polypeptides for detecting bovine coronavirus spike glycoprotein (S2), especially for detecting a polypeptide comprising the amino acid sequence of SEQ ID NO1: 1 or a fragment thereof. This detection preferably occurs through binding of the polypeptides of the present invention to the spike glycoprotein (S2). This binding can take place *in vitro* and/or *in vivo.* For example, the polypeptides of the present invention may be used in research laboratories in cell culture and FACS assays, Western Blot analyses or the like. For the detection purposes, the spike glycoprotein (S2)-binding polypeptides may be fused to other substances such as epitope tags, fluorescent molecules, enzymes and so on.

It is another object of the invention to provide a composition comprising the antigen-binding polypeptide of the present invention, particularly the antibody fragment and more particularly the single-chain antibody which specifically binds to bovine coronavirus spike glycoprotein (S2), and an acceptable excipient, carrier, buffer and/or stabilizer.

The antigen-binding polypeptides of the present invention may be degraded rapidly after oral application. Special attention has to be given to the high acidity in the stomach as well as the presence of secretory proteases, particularly in the oral cavity and in the small intestine.

Therefore the excipient, carrier, buffer and/or stabilizer preferably comprises a protein source, preferably BSA, milk powder and/or pea flour. The proteins may be added alone or as a protein mixture. Additionally or alternatively, the excipient, carrier, buffer and/or stabilizer may comprise methyl-cellulose.

Furthermore, the antigen binding peptides may be formulated as acid-resistant microparticles, or be packed into acid-resistant capsules, preferably gelatine capsules, which are preferably encased with a stabilizing liquid.

The present invention further extends to polynucleotides comprising a nucleotide sequence which encodes an antigen-binding polypeptide according to the invention, which specifically bind to bovine coronavirus spike glycoprotein (S2). The term "polynucleotide" as used herein is to be understood as meaning a double-stranded or single-stranded nucleic acid molecule, e.g. a DNA, cDNA, genomic DNA, RNA and/or mRNA molecule or the like. The nucleic acid molecule can be present either as the coding strand or as the complementary strand. The polynucleotide may be of a natural source or produced by gene technological or chemical processes and synthesis methods or may have been derived therefrom.

The individual nucleotide sequences and the consensus nucleotide sequences of VH CDR1-3 and VL CDR1-3 of the seven single-chain polypeptides DA6, DA10, DA11, DA25, DA30, DA31 and DA43 are depicted in the following tables 3-8. Nucleotides of the consensus sequences in parentheses are optional. Abbreviations for alternative nucleotides in the nucleotide sequences (underlined) are:
R: G or A
Y: T or C
M: A or C
K: G or T
S: G or C
W: A or T
B:T,GorC
D: A, T or G
H: A, T or C
V: A, G or C
N: any

**Table 3: Consensus and individual nucleotide sequences of VH CDR1 of the spike glycoprotein (S2)-binding polypeptides DA6, DA10, DA11, DA25, DA30, DA31 and DA43. The most often occurring common nucleotides are highlighted in bold letters.**

| | nucleotide sequence | SEQ ID No. |
|---|---|---|
| DA6 | **AAT TAC TTG ATA GAG** | 72 |
| DA30 | **AAT TAC TTG ATA GAG** | 72 |
| DA43 | **AAT TAC TTG ATA GAG** | 72 |
| DA31 | **AAT TAC TTG ATA GAG** | 72 |
| DA10 | G**A**C AC**C T**AT **ATA** C**A**C | 73 |
| DA11 | A**A**C **TA**T GCC **AT**G TCT | 74 |
| DA25 | A**A**C **TA**T GCC **AT**G TCT | 74 |

| consensus: | | |
|---|---|---|
| all | R**A**Y WMY KHB **AT**R BMB | 75 |
| DA6+30+43+31 | **AAT TAC TTG ATA GAG** | 72 |
| DA6+30+43+31+10 | R**A**Y WM**C T**WK **ATA** S**A**S | 76 |
| DA11+25 | A**A**C **TA**T GCC **AT**G TCT | 74 |
| DA6+30+43+31+11+25 | **AA**Y **TA**Y KYS **AT**R KMK | 77 |

**Table 4 :Consensus and individual nucleotide sequences of VH CDR2 of the spike glycoprotein (S2)-binding polypeptides DA6, DA10, DA11, DA25, DA30, DA31 and DA43. The most often occurring common nucleotides are highlighted in bold letters.**

| | nucleotide sequence | SEQ ID No. |
|---|---|---|
| DA6 | GTG ATT AAT CCT GGA AGT GGT GGT ACT AAC TAC AAT GAG AAG TTC AAG GGC | 78 |
| DA30 | GTG ATT AAT CCT GGA AGT GGT GGT ACT AAC TAC AAT GAG AAG TTC AAG GAC | 79 |
| DA43 | GTG ATT AAT CCT GGA AGT GGT GGT ACT AAC TAC AAT GAG AAG TTC AAG GGC | 78 |
| DA31 | GTG ATT AAT CCT GGA AGT GGT GGT ACT AAC TAC AAT GAG AAG TTC AAG GGC | 78 |
| DA10 | AGG ATT GAT CCT GCG ATT GGT AAT ACT AAA TAT GAC CCG AAG TTC CAG GGC | 80 |
| DA11 | TCC ATT AAT AGT GGT --- GGT TAC ACC TAC TAT CCA GAC AGT GTG AAG GGC | 81 |
| DA25 | TCC ATT ACT AGT GGC --- GGT ATT ACC TAC TAT CCA GAC AGT GTG AAG GGC | 82 |

| consensus: | | |
|---|---|---|
| all | DBS ATT RMT MST GSN (AKT) GGT DDY ACY WAM TAY VMH SMS ARK KTS MAG GRC | 83 |
| DA6+30+43+31 | GTG ATT AAT CCT GGA AGT GGT GGT ACT AAC TAC AAT GAG AAG TTC AAG GRC | 84 |
| DA6+30+41+31+10 | RKG ATT RAT CCT GSR AKT GGT RRT ACT AAM TAY RAY SMG AAG TTC MAG GRC | 85 |
| DA11+25 | TCC ATT AMT AGT GGY GGT WWY ACC TAC TAT CCA GAC AGT GTG AAG GGC | 86 |
| DA6+30+43+31+11+25 | KYS ATT AMT MST GGH (AGT) GGT DDY ACY WAC TAY MMW GAS ARK KTS AAG GRC | 87 |

**Table 5:Consensus and individual nucleotide sequences of VH CDR3 of the spike glycoprotein (S2)-binding polypeptides DA6, DA10, DA11, DA25, DA30, DA31 and DA43. The most often occurring common nucleotides are highlighted in bold letters.**

| | nucleotide sequence | SEQ ID No. |
|---|---|---|
| DA6 | TAT ACC CTC TAC TAT GGT AAC CCT TAT TAC TAT GCT ATG GAC TAC | 88 |
| DA30 | TTT ACC CTC TAT CAT GGT AAC CCT TAT TAC TAT GCT ATG GAC TAC | 89 |
| DA43 | TAT ACC CTC TAC TAT GGT AAC CCT TAT TAC TAT GCT ATG GAC TAC | 88 |
| DA31 | TAT ACC CTC TAC TAT GGT AAC CCT TAT TAC TAT GCT ATG GAG TAC | 90 |
| DA10 | AGG AAT --- TTC TAT --- GCT ATG GAC TAC | 91 |
| DA11 | GGC GGA CGA CCC CTT TAC TTT --- --- GAC TAC | 92 |
| DA25 | GGC GGA CGG CCC CTT TAC TTT --- --- GAC TAC | 93 |

| consensus: | | |
|---|---|---|
| all | (TWT ACC CTC TAY) (BRY) RGD MRN (CCY) YWY TAY (TWT) (GCT ATG) GAS TAC | 94 |
| DA6+30+43+31 | TWT ACC CTC TAY YAT GGT AAC CCT TAT TAC TAT GCT ATG GAS TAC | 95 |
| DA6+30+43+31+10 | (TWT ACC CTC TAY YAT) RGK AAY (CCT) TWY TAY (TAT) GCT ATG GAS TAC | 96 |
| DA11+25 | GGC **GGA** CGR **CC**C CT**T TAC T**T**T GAC TAC** | 97 |
| DA6+30+43+31+11+25 | (TWT ACC CTC TAY) BRY GGW MRV CCY YWT TAC TWT (GCT ATG) GAS TAC | 98 |

**Table 6: Consensus and individual nucleotide sequences of VL CDR1 of the spike glycoprotein (S2)-binding polypeptides DA6, DA10, DA11, DA25, DA30, DA31 and DA43. The most often occurring common nucleotides are highlighted in bold letters.**

| | nucleotide sequence | SEQ ID No. |
|---|---|---|
| DA6 | CGC TCA AGT ACT GGG GCT GTT ACA ACT AGT AAC TAT GCC AAC | 99 |
| DA30 | CGC TCA AGT ACT GGG GCT GTT ACA ACT AGT AAC TAT GCC AAC | 99 |
| DA43 | CGC TCA AGT ACT GGG GCT GTT ACA ACT AGT AAC TAT GCC AAC | 99 |
| DA31 | AGG GCA AGT CAG GAC --- --- --- ATT AGC AAT TAT TTA AAC | 100 |
| DA10 | AGT GCA AGT CAG GGC --- --- --- ATT AGC AAT TAT TTA AAC | 101 |
| DA11 | AGT GCC AGC TCA AGT --- GTA --- --- AGT --- TAC ATG CAC | 102 |
| DA25 | AGT GCC AGC TCA AGT --- GTA --- --- AGT --- TAC ATG CAC | 102 |

| consensus: | | |
|---|---|---|
| all | MGB KCM AGY HMD RRB (GCT) (GTW) (ACA) (AYT) AGY (AAY) TAY DYV MAC | 103 |
| DA6+30+43 | CGC TCA AGT ACT GGG GCT GTT ACA ACT AGT AAC TAT GCC AAC | 99 |
| DA31+10+11+25 | AGK GCM AGY YMR RRY (GTA) (ATT) AGY (AAT) TAY WTR MAC | 104 |
| DA31+10 | A**G**K G**CA AGT** CAG **G**RC **A**T**T AG**C **AA**T **TAT** TTA **AAC** | 105 |
| DA11+25 | A**G**T G**C**C **AG**C T**C**A A**G**T **GT**A **AGT TA**C ATG C**AC** | 102 |
| DA6+30+41+31+10 | MGB KCA AGT MMK GRS (GCT GTT ACA) AYT AGY AAY TAT KYM AAC | 106 |
| DA6+30+43+11+25 | MGY KCM AGY WCW RGK (GCT) GTW (ACA ACT) AGT (AAC) TAY RYS MAC | 107 |

**Table 7: Consensus and individual nucleotide sequences of VL CDR2 of the spike glycoprotein (S2)-binding polypeptides DA6, DA10, DA11, DA25, DA30, DA31 and DA43. The most often occurring common nucleotides are highlighted in bold letters.**

| | nucleotide sequence | SEQ ID No. |
|---|---|---|
| DA6 | **GGT ACC AAC AAC CGA** C**CT CCA** | 108 |
| DA30 | **GGT ACC AAC AAC CGA GCT CCA** | 109 |
| DA43 | **GGT ACC AAC AAC CGA GCT CCA** | 109 |
| DA31 | TAC **AC**A TCA **A**GA TT**A** CAC T**CA** | 110 |
| DA10 | TAC **AC**A TCA **A**GT TT**A** CAC T**CA** | 111 |
| DA11 | **G**AC **AC**A TCC **AA**A **C**TG **GCT** T**C**T | 112 |
| DA25 | **G**AC **AC**A TCC **AA**A **C**TG **GCT** T**C**T | 112 |

| consensus: | | |
|---|---|---|
| all | KRY **AC**M WMM **A**RH YKR SMY Y**C**W | 113 |
| DA6+30+43 | **GGT ACC AAC AAC CGA** S**CT CCA** | 114 |
| DA31+10+11+25 | KAC **AC**A TCM **A**RW YTR SMY T**C**W | 115 |
| DA31+10 | TAC **AC**A TCA **A**GW TT**A** CAC T**CA** | 116 |
| DA11+25 | **G**AC **AC**A TC**C AA**A **C**TG **GCT** T**C**T | 112 |
| DA6+30+43+31+10 | KRY **AC**M WMM **A**RH YK**A** SMY YC**A** | 117 |
| DA6+30+43+11+25 | **G**RY **AC**M WM**C AA**M **C**KR S**CT** Y**C**W | 118 |

**Table 8: Consensus and individual nucleotide sequences of VL CDR3 of the spike glycoprotein (S2)-binding polypeptides DA6, DA10, DA11, DA25, DA30, DA31 and DA43. The most often occurring common nucleotides are highlighted in bold letters.**

| | nucleotide sequence | SEQ ID No. |
|---|---|---|
| DA6 | **GCT CTA TGG TAC AGC AAC CAT TGG GTG** | 119 |
| DA30 | **GCT CTA TGG TAC AGC AAC CA**C **TGG GTG** | 120 |
| DA43 | **GCT CTA TGG T**T**C AGC AAC CAT TGG GTG** | 121 |
| DA31 | CAA **C**AG G**G**T A**A**T **A**CG CTT **C**CG **TGG** AC**G** | 122 |
| DA10 | CAG **C**AG **T**AT AGT **A**AG CTT **C**C**T** C**GG** AC**G** | 123 |
| DA11 | CAG **C**AG **TGG** AGT **AG**T **AAC C**CA CCA AC**G** | 124 |
| DA25 | CAG **C**AG **TGG** AGT **AG**T **AAC C**CG CTC AC**G** | 125 |

| consensus: | | |
|---|---|---|
| all | SMD **C**WR KRK WDY **A**VB MWY **C**MN YBV RY**G** | 126 |
| DA6+30+43 | **GCT CTA TGG T**W**C AGC AAC CA**Y **TGG GTG** | 127 |
| DA31+10+11+25 | CAR **C**AG KRK ART **A**VK MWY **C**CD YBV AC**G** | 128 |
| DA31+10 | CAR **C**AG KRT ART **A**MG CTT **C**CK Y**GG** AC**G** | 129 |
| DA11+25 | CAG **C**AG **TGG** AGT **AG**T **AAC C**CR CYM AC**G** | 130 |
| DA6+30+43+31+10 | SMD **C**WR KRK WDY **A**VS MWY **C**MB Y**GG** RY**G** | 131 |
| DA6+30+43+11+25 | SMK **C**WR **TGG** WDY **AG**Y **AAC C**MN YBV RY**G** | 132 |

Thus, in a preferred embodiment, the polynucleotide of the present invention comprises the nucleotide sequence encoding the antigen-binding portion of an antibody heavy chain variable region, which comprises a VH CDR 1, which is encoded by the nucleotide sequence depicted in SEQ ID NO: 75, more preferably by the sequences depicted in SEQ ID NOs: 72, 73 and 74. Other preferred consensus nucleotide sequences are depicted in SEQ ID NOs: 76 and 77. Most preferred for encoding VH CDR1 are the individual nucleotide sequences depicted in SEQ ID NOs: 72, 73 and 74. Also preferred in the present invention is a polynucleotide which comprises a nucleotide sequence encoding the VH CDR1 depicted in the amino acid sequences SEQ ID NOs: 16-21, preferably 16-18.

In another preferred embodiment, the polynucleotide of the present invention comprises the nucleotide sequence encoding the antigen-binding portion of an antibody heavy chain variable region, which comprises a VH CDR 2, which is encoded by the nucleotide sequence depicted in SEQ ID NO: 83, more preferably by the sequences depicted in SEQ ID NOs: 80, 84 and 86. Other preferred consensus nucleotide sequences are depicted in SEQ ID NOs: 85 and 87. Most preferred for VH CDR2 are the individual nucleotide sequences depicted in SEQ ID NOs: 78, 79, 80, 81 and 82. Also preferred in the present invention is a polynucleotide which comprises a nucleotide sequence encoding the VH CDR2 depicted in the amino acid sequences SEQ ID NOs: 22-31, preferably 22-26.

In another preferred embodiment, the polynucleotide of the present invention comprises the nucleotide sequence encoding the antigen-binding portion of an antibody heavy chain variable region, which comprises a VH CDR 3, which is encoded by the nucleotide sequence depicted in SEQ ID NO: 94, more preferably by the sequences depicted in SEQ ID NOs: 91, 95 and 97. Other preferred consensus nucleotide sequences are depicted in SEQ ID NOs: 96 and 98. Most preferred for VH CDR3 are the individual nucleotide sequences depicted in SEQ ID NOs: 88, 89, 90, 91, 92 and 93. Also preferred in the present invention is a polynucleotide which comprises a nucleotide sequence encoding the VH CDR3 depicted in the amino acid sequences SEQ ID NOs: 32-40, preferably 32-36.

In another preferred embodiment, the polynucleotide of the present invention comprises the nucleotide sequence encoding the antigen-binding portion of an antibody heavy chain variable region, which comprises a VL CDR 1, which is encoded by the nucleotide sequence depicted in SEQ ID NO: 103, more preferably by the sequences depicted in SEQ ID NOs: 99, 102 and 105. Other preferred consensus nucleotide sequences are depicted in SEQ ID NOs: 104, 106 and 107. Most preferred for VL CDR1 are the individual nucleotide sequences depicted in SEQ ID NOs: 99, 100, 101 and 102. Also preferred in the present invention is a polynucleotide which comprises a nucleotide sequence encoding the VL CDR1 depicted in the amino acid sequences SEQ ID NOs: 41-49, preferably 41-44.

In another preferred embodiment, the polynucleotide of the present invention comprises the nucleotide sequence encoding the antigen-binding portion of an antibody heavy chain variable region, which comprises a VL CDR 2, which is encoded by the nucleotide sequence depicted in SEQ ID NO: 113, more preferably by the sequences depicted in SEQ ID NOs: 112, 114 and 116. Other preferred consensus nucleotide sequences are depicted in SEQ ID NOs: 115, 117 and 118. Most preferred for VL CDR2 are the individual nucleotide sequences depicted in SEQ ID NOs: 108, 109, 110, 111 and 112. Also preferred in the present invention is a polynucleotide which comprises a nucleotide sequence encoding the VL CDR1 depicted in the amino acid sequences SEQ ID NOs: 50-58, preferably 50-53.

In another preferred embodiment, the polynucleotide of the present invention comprises the nucleotide sequence encoding the antigen-binding portion of an antibody heavy chain variable region, which comprises a VL CDR 3, which is encoded by the nucleotide sequence depicted in SEQ ID NO: 126, more preferably by the sequences depicted in SEQ ID NOs: 127, 129 and 130. Other preferred consensus nucleotide sequences are depicted in SEQ ID NOs: 128, 131 and 132. Most preferred for VL CDR3 are the individual nucleotide sequences depicted in SEQ ID NOs: 119, 120, 121, 122, 123, 124 and 125. Also preferred in the present invention is a polynucleotide which comprises a nucleotide sequence encoding the VL CDR1 depicted in the amino acid sequences SEQ ID NOs: 59-71, preferably 59-64.

The polynucleotides may also comprise sequences which hybridize to a complementary strand of the above mentioned nucleotide sequences or are a degenerate of the above mentioned nucleotide sequences.

In another preferred embodiment, the polynucleotide comprising a nucleotide sequence which encodes an antigen-binding polypeptide according to the invention, which specifically binds to bovine coronavirus spike glycoprotein (S2), is selected from the group consisting of
a) polynucleotides comprising a nucleotide sequence which encodes the amino acid sequence identified in SEQ ID NOs: 2, 3, 4, 5, 6, 7 and 8,
b) polynucleotides comprising the nucleotide sequence identified in SEQ ID NOs: 9, 10, 11, 12, 13, 14 and 15,
c) polynucleotides comprising a nucleotide sequence which hybridizes to a complementary strand of the nucleotide sequence of a) or b),
d) polynucleotides comprising a nucleotide sequence which is degenerate to a nucleotide sequence of c),
e) polynucleotides which represent a derivative, analogue, part or fragment of a nucleotide sequence of a), b), c) or d).

The terms "to hybridize" or "hybridization" describe the process by which a single-stranded polynucleotide enters into base-pairing with a complementary polynucleotide strand. In the context of the present invention the term "hybridization" means a hybridization under conventional hybridization conditions, preferably under stringent conditions, for example as described in Sambrook et al. (1989), Molecular Cloning: A Laboratory Manual, 2. Ed., Cold Spring Harbour Laboratory Press, Cold Spring Harbour, NY.

Suitable stringent conditions include salt solutions of about 0.9 molar at temperatures of 35°C to 65°C. Stringent hybridization conditions preferably comprise the following conditions:

| | |
|---|---|
| Hybridization buffer: | 7% SDS |
| | 250 mM NaCl |
| | 250 mM K-phosphate buffer pH 7.0 |
| | 1 mM EDTA |
| Hybridization temperature: | 58°C to 60°C |
| Hybridization time: | overnight |
| Washing buffer: | I 2 x SSC 0.1% SDS |
| | II 0.2 x SSC 0.1% SDS |
| Washing temperature and time: | each 2 x 30 min at 55°C to 60°C |

The degeneration of the genetic code offers one skilled in the art among other things the possibility of adapting the nucleotide sequence to the codon preference of the target host cell, thereby optimizing the expression of the desired antigen-binding polypeptide of the present invention.

The above-mentioned polynucleotides also comprise fragments, derivatives, analogues or parts of the polynucleotide sequences described above, which code for a polypeptide, particularly an antibody fragment, and especially a single-chain antibody, having the biological characteristics of a polypeptide which specifically binds to bovine coronavirus spike glycoprotein (S2). The fragments, derivatives, analogues or parts may also be both naturally occurring variations or mutations, wherein these mutations may have occurred naturally or have been introduced e.g. by targeted mutagenesis. Moreover, the variations can further comprise synthetic sequences.

The polypeptide being encoded by the fragments, derivatives, analogues or parts of the polynucleotide sequences described above is preferably characterized by "conservative" amino acid substitutions, for instance substitution of one hydrophobic residue for another or the substitution of one polar residue for another. At certain positions non-conservative substitutions are allowable.

The term "fragments" is to be understood as parts of the polynucleotide sequence that are sufficiently long to encode one of the described polypeptides. The term "derivative" in this context means that the sequences differ from the polynucleotide sequences described above at one or several position(s), but have a high degree of homology to these sequences. Homology here means a sequence identity of at least 40%, particularly an identity of at least 60% or 70%, preferably of at least 80%, 82%, 84%, 86% or 88%, and particularly preferably of at least 90%, 92%, 94%, 96% or 98%. Variations from the nucleotide sequences described above may be caused, for example, by deletion, substitution, insertion or recombination.

The proteins encoded by these polynucleotide sequences show a sequence identity to the amino acid sequences identified in SEQ ID NOs: 2, 3, 4, 5, 6, 7 and 8, respectively, of at least 40%, particularly an identity of at least 60% or 70%, preferably of at least 80%, 82%, 84%, 86% or 88%, and particularly preferably of at least 90%, 92%, 94%, 96% or 98%. The differences to the above described polynucleotide sequences may be the result of, for example, deletion, substitution, insertion or recombination.

In order to determine the percentage of homology (= identity) between two amino acid or nucleotide sequences, the two sequences are aligned, and the amino acids or nucleotides at each position are compared. If one position within the sequences is occupied by the same amino acid or the same nucleotide, then the molecules at this position are homologous (= identical). The percentage of homology between the two sequences is a function of the number of common positions that are identical (i.e. homology = number of identical positions per total number of positions x 100).

The homology is calculated over the total amino acid or nucleotide sequence area. In order to compare different sequences a variety of programs based on different algorithms are available to the person skilled in the art. The algorithms of Needleman and Wunsch or Smith and Waterman provide especially reliable results. For the sequence alignments and comparisons, the programs "PileUp" (J. Mol. Evolution., 25, 351-360, 1987, Higgins et al., CABIOS, 5 1989: 151-153) or "Gap" and "BestFit" [Needleman and Wunsch (J. Mol. Biol. 48; 443-453 (1970) and Smith and Waterman (Adv. Appl. Math. 2; 482-489 (1981)], which are enclosed in the GCG Software-Packet [Genetics Computer Group, 575 Science Drive, Madison, Wisconsin, USA 53711 (1991)], can be used. The sequence homology values mentioned above as percentages can be determined by means of the "Gap" program over the total sequence area with the following adjustments: Gap Weight: 50, Length Weight: 3, Average Match: 10.000 und Average Mismatch: 0.000. These adjustments can be used as standard adjustments for sequence homology analyses.

The polynucleotides comprising the nucleotide sequence identified in SEQ ID NOs: 9, 10, 11, 12, 13, 14 or 15 may be altered by replacing the linker encoding nucleotide sequence (here: AAG CTT GAA GAA GGT GAA TTT TCA GAA GCA CGC GT, SEQ ID NO: 137) with any other desired linker encoding nucleotide sequence. Further, the nucleotide sequences encoding the heavy chain and the nucleotide sequences encoding the light chain of SEQ ID NOs: 9, 10, 11, 12, 13, 14 or 15 may be combined with one another in any conceivable way.

Preferably, the polynucleotide of the present invention comprising a nucleotide sequence encoding the antigen-binding polypeptide, which specifically binds to bovine coronavirus spike glycoprotein (S2), is under control of regulatory sequences which provide for a specific transcription. This can be achieved by conventional cloning methods (e.g. Sambrook et al. 1989, Molecular Cloning: A Laboratory Manual, 2. Ed., Cold Spring Harbour Laboratory Press, Cold Spring Harbour, NY). Thus, the present invention further provides a recombinant polynucleotide molecule, which comprises
a) a promoter region,
b) a polynucleotide according to the invention as described above, which is operatively linked to the promoter region, and
c) optionally, regulatory sequences operatively linked thereto, which may act as transcription, termination and/or polyadenylation signals, enhancer sequences and/or sequences coding for leader signals.

Using routine molecular biological techniques (see for example Sambrook *et al.,* vide supra) it is possible to prepare and to produce the desired constructs for the transformation of any prokaryotic or eukaryotic cell. Methods for cloning, mutagenesis, sequence analysis, restriction analysis and other biochemical/molecular biological methods are well known to the person skilled in the art and are conventionally used for gene technological manipulation.

Thus, not only may suitable polynucleotide constructs containing the desired fusion of a promoter sequence and an antigen-binding polypeptide sequence of the present invention and optionally further regulatory sequences be produced, but rather the person skilled in the art may further, if desired, introduce various mutations, deletions, fusions or insertions of natural or artificial nucleotide sequences into the nucleotide sequence encoding the antigen-binding polypeptide according to the invention using routine techniques. For the fusion of the constructs with each other adapters or linkers may be attached to the constructs where necessary. Additionally, appropriate restriction sites may be provided or removed using enzymatic and other manipulation techniques. This may result in the synthesis of a protein with altered biological properties, e.g. stronger binding properties (affinity, avidity, time period) or higher stability, e.g. temperature stability, pH stability and/or stability against protease cleavage, or resulting in the synthesis of proteins with altered expression properties, e.g. at the transcription and/or translation level, or with altered purification capacities.

The present invention also refers to vectors comprising the polynucleotide which encodes the antigen-binding polypeptide of the present invention, particularly the antibody fragment and more particularly the single-chain antibody, which specifically binds to bovine coronavirus spike glycoprotein (S2). The term "vector" describes a polynucleotide which is used for introducing exogenous polynucleotides into host cells. A vector contains a nucleotide sequence which encodes one or more polypeptides. Preferably the vector of the present invention is a phage display vector, a bacterial expression vector, a yeast expression vector, a fungus expression vector, an algae expression vector or a plant expression vector. Vectors which are able to control the expression of the genes which they contain are termed "expression vectors".

In order to prepare the introduction of foreign genes in higher plants a variety of vectors are available which contain replicating signals for *E. coli* and a marker gene for selecting transformed bacterial cells. Examples for such vectors are pBR322, pUC series, M13mp series, pACYC184, pBlueSfi and the like. The desired sequence may be introduced in the vector at a suitable restriction site. The resulting plasmid is then used for the transformation of *E. coli* cells. Transformed *E. coli* cells are cultivated in a suitable medium and then harvested and lysed, and the plasmid is recovered. In order to characterize the produced plasmid DNA in general restriction analysis, gel electrophoresis and further biochemical and molecular biological methods are used as analytic method. After each manipulation step the plasmid DNA may be cleaved and the obtained DNA fragments may be linked to other DNA sequences.

Several known techniques are available for introducing DNA in a plant host cell, and the person skilled in the art will not have any difficulties in selecting a suitable method. These techniques comprise the transformation of plant cells with T DNA by use of *Agrobacterium tumefaciens* or *Agrobacterium rhizogenes* as the transforming agent, the fusion of protoplasts, the direct gene transfer of isolated DNA into protoplasts, the electroporation of DNA, the introduction of DNA by means of the biolistic method as well as further possibilities.

During the injection and electroporation of DNA into plant cells no specific requirements for the used plasmids are necessary per se. The same is true for the direct gene transfer. Plain plasmids such as pUC and pBlueScript derivatives may be used. The presence of a selectable marker gene is necessary if entire plants are to be regenerated from such transformed cells. The person skilled in the art is familiar with these gene selection markers and will not have any problems in selecting a suitable one.

Further DNA sequences may be required depending on the introduction method for desired genes into the plant cell. If, for example, the Ti or Ri plasmid is used for the transformation of the plant cell, at least the right border, however more often both, the right and the left border of the T DNA in the Ti or Ri plasmid, has to be linked as flanking region to the genes to be introduced.

If agrobacteria are used for the transformation, the DNA to be introduced has to be cloned into special plasmids, either into an intermediate or into a binary vector. Intermediate vectors may be integrated into the Ti or Ri plasmid of the agrobacteria by homologous recombination via sequences which are homologous to sequences in the T DNA. This also contains the *vir* region which is required for T DNA transfer. Intermediate vectors are not able to replicate in agrobacteria. With the aid of a helper plasmid the intermediate vector may be transferred to *Agrobacterium tumefaciens* (conjugation). Binary vectors are able to replicate in *E.coli* as well as in agrobacteria. They contain a selection marker gene, and a linker or polylinker framed by the right and left T DNA border region. They may be transformed directly into agrobacteria. The agrobacterial host cell should contain a plasmid with a *vir* region. The *vir* region is required for the transfer of the T DNA into the plant cell. Additional T DNA may be present. Such a transformed agrobacterial cell is used for the transformation of plant cells.

The use of T DNA for the transformation of plant cells has been studied intensively, and has been sufficiently described in generally known reviews and plant transformation manuals.

For transfer of the DNA into the plant cell, plant explants may be cultivated for this purpose together with *Agrobacterium tumefaciens or Agrobacterium rhizogenes.* From the infected plant material (e.g. leaf pieces, stem segments, roots, but also protoplasts or suspension cultivated plant cells) whole plants may be regenerated in a suitable medium which may contain antibiotics or biocides for selection of transformed cells. Regeneration of plants may take place according to conventional regeneration methods by use of known growth media. The resulting plants may be analyzed for the presence of the introduced DNA. Other possibilities for the introduction of foreign DNA by use of biolistic methods or protoplast transformation are well known, and as have been described extensively.

Once the introduced DNA has been integrated into the plant cell genome it is generally stable there and is maintained in the progeny of the originally transformed cell as well. Usually it contains a selection marker which allows the selection of transformed cells from those lacking the introduced DNA. This selection marker endows the transformed plant cells with resistance to a biocide or an antibiotic, such as kanamycin, G418, bleomycin, hygromycin, methotrexate, glyphosate, streptomycin, sulfonylurea, gentamycin or phosphinotricin and others. But also nutritive markers and screening markers (such as GFP, green fluorescent protein) are being used as alternatives to antibiotics resistance markers.It is also possible to perform this selection procedure without any selection marker, however this requires a quite high screening expenditure.

The resulting plants may be cultivated normally, and may be crossbred with plants having the same transformed hereditary disposition or other predispositions. The resulting hybrids will have the pertinent phenotype characteristics. Seeds may be obtained from the plant cells.

Two or more generations should be generated to ensure that the phenotypic feature is stably maintained and inherited. Additionally, seeds should be harvested to verify the maintenance of the respective phenotype or other features.

In a preferred embodiment, the vector comprising the polynucleotide which encodes the antigen-binding polypeptide of the present invention, particularly the antibody fragment and more particularly the single-chain antibody, which specifically binds to bovine coronavirus spike glycoprotein (S2), contains specific regulatory elements, either constitutive or inducible (which may be either by biotic or abiotic stimuli), especially promoters, specific for the expression of the cloned construct in the prokaryotic or eukaryotic host cell, e.g. in bacteria, yeast or plant cells. For the expression of the polypeptides of the present invention in a plant cell or a microorganism cell, in principal any promoter can be considered which is active in the respective host cells. The promoter may be homologous or heterologous to the cell to be transformed.

For plant expression, the promoter is preferably tissue specific, but it may also be development-specific. The promoter may e.g. be active in tissues that are used as fodder such as seed, leaf and fruit tissues. The most preferred promoters for plant expression are seed specific promoters.

Useful promoters for seed specific expression are the USB promoter, preferably the long (Zakharov et al. 2004, J Exp Bot., Jul;55(402):1463-71. Epub 2004 Jun 4) or the short USB promoter (Bäumlein et al. 1991, Mol. Gen. Genet. 225:459-467). Other promoters which can be used in the present invention are the NOS-promoter, the Vicilin promoter, the Phaseolin promoter, the Actin promoter, the Arcelin promoter, the Hordein promoter (Brandt et al. 1985, Carlsberg Res. Commun. 50:333-345) and the Napin promoter, the ACP promoter as well as the FatB3- and FatB4 promoters, the 35S RNA promoter of the Cauliflower Mosaic Virus, the Ubiquitin promoter from maize, or the LeB4 promoter. The following publication summarizes most of the useful promoters: Zakharov et al. 2004, J Exp Bot., Jul;55(402):1463-71. Epub 2004 Jun 4.

Leader sequences may also be introduced into the vector, e.g. in order to achieve secretion of the polypeptides of the present invention into the medium. For example, the amino acid sequence "EAYA" is a cleavage site at the C terminus of a leader sequence. The "KDEL" sequence at the C terminus of a polypeptide sequence is an endoplasmatic reticulum (ER) retention signal in plants.

Useful promoters for the expression in microorganisms are e.g. the Lac Operon/promoter, the T3/T7 promoter, and the AOX promoter.

These promoters are well known to a person skilled in the art of molecular biology. In any case the skilled person can find suitable promoters in the literature, e.g. in relevant scientific journals and gene databases, or can isolate them from any desired organism using standard methods.

Another aspect of the present invention is to provide prokaryotic or eukaryotic host cells which comprise a polynucleotide encoding an antigen-binding polypeptide of the present invention or a vector comprising said polynucleotide. Preferably, the host cells are stably or transiently transformed with the above-described vectors of the present invention. Still another aspect of the present invention is to provide transgenic plants or parts thereof as well as microorganisms containing an above-described polynucleotide or an above-described vector of the present invention.

Preferably, the host cells or the plants or plant parts or microorganisms of the present invention express and synthesize the antigen-binding polypeptide of the present invention. These host cells may be any prokaryotic or eukaryotic cells, preferably microorganismic cells or plant cells, more preferably bacterial, yeast, fungus, algae and plant cells. Most preferred among the microorganismic cells are *Escherichia coli* cells, Streptomyces cells, *Pichia pastoris* cells, *Schizosaccharomyces* cells, especially *Schizosaccharomyces pombe* cells, Aspergillus cells, and Chlamydomonas cells.

The plant cells and plants of the present invention may be in principle any plant cells or plants. Preferably, it is a monocotyledonous or dicotyledonous crop plant and more preferably a fodder plant. The most preferred plants are a *Nicotiana spec.,* especially *Nicotiana benthamiana* and *Nicotiana tabacum, Pisum sativum, Hordeum vulgare, Zea mays, Cucumis sativus* and *Linum usitatissimum* and *Brassica napus.* Other plants which may be used are e.g. poppy, olive, cocoa, barley, wheat, almond, sesame, mustard, castor oil plant, sunflower, soybean, peanut, coconut, turnip seed, cotton, rice and oil palm trees.

The invention also relates to propagation material and agricultural products of the plants according to the invention, for example fruits, seeds, tubers, rootstocks, seedlings, cuttings and the like.

Further, the invention relates to the use of the plants, plant parts or microorganisms of the present invention as fodder for mammals, especially for cattle.

Another object of the invention is to provide a method of producing an antigen-binding polypeptide of the present invention, particularly an antibody fragment and more particularly a single-chain antibody, which specifically binds to coronavirus spike glycoprotein (S2), comprising:
- providing an expression vector which comprises a polynucleotide encoding said antigen-binding polypeptide, and
- expressing said vector in a prokaryotic or eukaryotic host cell.

The expression vector, the polynucleotide and the host cell used for this method are described above.

### EXAMPLES

The following examples are intended to illustrate the present invention and are in no way to be understood as limiting.

### Example 1: Generation of a phage display library

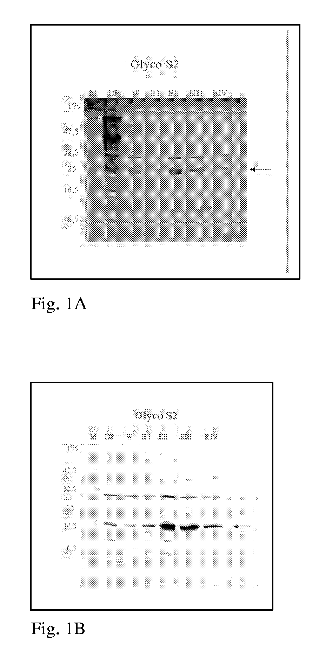

The nucleic acid encoding bovine coronavirus spike glycoprotein (S2) (cf. NCBI accession number D00662, comprising both S1 and S2) was cloned into a pET16b Vector and expressed in *E.coli* BL21 cells. Cells were harvested by centrifugation (15min 3000xg), incubated in binding buffer (20mM Tris, 0.5M NaCl, 5mM imidazol, pH=7.9) sonicated and centrifuged again (30min 50000g). The antibody was purified via HIS-tag using IMAC (Immobilized Metal ion Affinity Chromatography). Both fractions were incubated for 2hrs with Ni-NTA Agarose, washed (20mM Tris-HCl, 1M NaCl, 5mM imidazol, pH 7,9) and the pure protein was eluted (20mM Tris-HCl, 1M NaCl, 500mM imidazol, pH 7,9) and then separated on an SDS gel (fig. 1A; molecular weight: 24,3 kDa). The gel was blotted on a Nitrocellulose membrane. The membrane was blocked one hour at room temperature (RT) in Rotiblock and afterwards incubated with the primary anti-His antibody (Sigma H1029) diluted 1:3000 in 1 % BSA-TBS / 0,05% Tween for one hour at RT. After washing three times with TBS / 0,05% Tween the membrane was incubated with the secondary anti-mouse-AP-conjugated antibody (Sigma A4312) diluted 1:30000 in 1 % BSA-TBS / 0,05% Tween for one hour at RT. After washing three times with TBS / 0,05% Tween and two times five min with TBS the membrane was developed with MBT/BCIP (fig. 1B).

Mice were immunized with this antigen. Blood samples showed high antibody titers in the antigen ELISA. The spleens of the mice were prepared and RNA isolated therefrom. After mRNA purification single stranded DNA was prepared. DNA quality was tested by PCR amplification of the immunoglobulin genes. An aliquot of the cDNA was cloned into a phage display vector. The phage display library contained recombinant antibody fragments (scFv = single chain fragment variable).

For library screening the spike glycoprotein (S2) antigen was immobilized on Maxisorp immunoplates and the phage display library was cycled three times for binding selection. Wells were blocked with BSA. Clones were selected randomly and assayed using a phage ELISA to compare binding to target antigen (spike glycoprotein (S2)) and several non-relevant proteins for preventing non-specific binding. Positive clones (only binding to spike glycoprotein (S2)) were further analyzed by restriction analysis, sequencing, Western Blot and ELISA. The following clones were isolated (table 9):

**Table 9: scFv antibodies obtained from a screening with coronavirus spike glycoprotein (S2). "Screened clones" are the clones which were analyzed by ELISA (384). 56 gave an ELISA signal up to 0,8 and 274 an ELISA signal up to 0,2. From the 384 clones analyzed by ELISA only 96 were further analyzed (protein expression, Western Blot, DNA extraction, restriction analysis). Unique scFv are scFv which have the same amino acid sequence. Max. 3 amino acids (10 amino acids) are different.**

| antigen | nomenclature | screened clones | positive clones ELISA >0.2 (>0.8) | analyzed clones | unique scFv > 3 amino acids (>10 amino acids) | in cell culture positive tested medium (strong) |
|---|---|---|---|---|---|---|
| Glyco (S2) Coronavirus | Daxx | 384 | 274(56) | 96 | 20 (15) | 7 (4) |

Four of the isolated scFv antibodies against spike glycoprotein (S2) showed a strong neutralizing effect (DA10, DA30, DA31 and DA43). Among the antibodies tested "medium" are DA6, DA11 and DA25.

For the ELISA assay, Maxisorp-plates (Nunc) were coated over night at 4°C with 1 µg / well Glyco (S2) antigen (in PBS pH 7,4). Plates were washed three times with PBS and blocked two hours at room temperature (RT). After three additional washes the BA11 antibody was added for two hours. Plates were washed once with PBS/0,05%Tween and four times with PBS before adding the primary anti-His antibody (Sigma H1029) diluted 1:3000 in 2 % BSA-PBS for one hour at RT. After washing once with PBS/0,05%Tween and four times with PBS the secondary anti-mouse-HRP-conjugated antibody (Sigma A4312) diluted 1:8000 in 2% BSA-PBS was incubated for one hour at RT. Plates were washed again once with PBS/0,05%Tween and four times with PBS before TMB/H₂O₂ was added. After 30min the reaction was stopped by adding 1M sulfuric acid and the OD₄₅₀ₙₘ was measured.

### Example 2: Expression of scFv antibodies

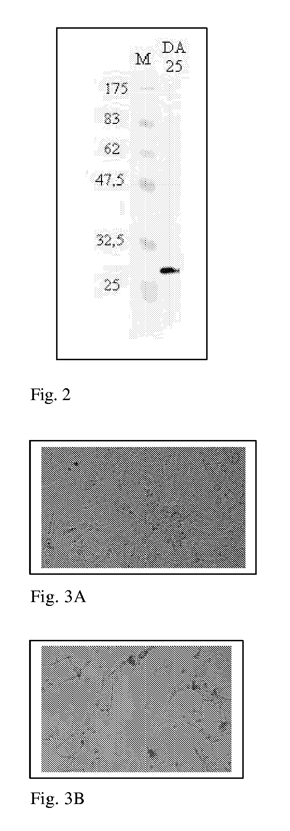

As expression systems (production systems) for the scFv antibodies *E. coli* was used. The DA25 sequence was cloned into the pET16b vector (Novagen) and transformed into BL21 cells (Stratagene). Cells were cultured until OD₆₀₀= 0,6 at 37°C in LB-medium. After induction with 100µM IPTG cells were grown for one hour at 37°C in LB-medium resulting in the expression of the DA25 construct. Cells were harvested by centrifugation, lysed in a sample loading buffer, and the proteins were separated on a 12% SDS-gel. The gel was blotted on a Nitrocellulose membran. The membrane was blocked one hour at room temperature (RT) in Rotiblock and afterwards incubated with the primary anti-His antibody (Sigma H1029) diluted 1:3000 in 1 % BSA-TBS/0,05%Tween for one hour at RT. After washing three times with TBS/0,05%Tween the membrane was incubated with the secondary anti-mouse-AP-conjugated antibody (Sigma A4312) diluted 1:30000 in 1 % BSA-TBS/0,05%Tween for one hour at RT. After washing three times with TBS/0,05%Tween and two times five min with TBS the membrane was developed with MBT/BCIP (fig. 2).

### Example 3: In vitro testing of antibodies (virus neutralization test)

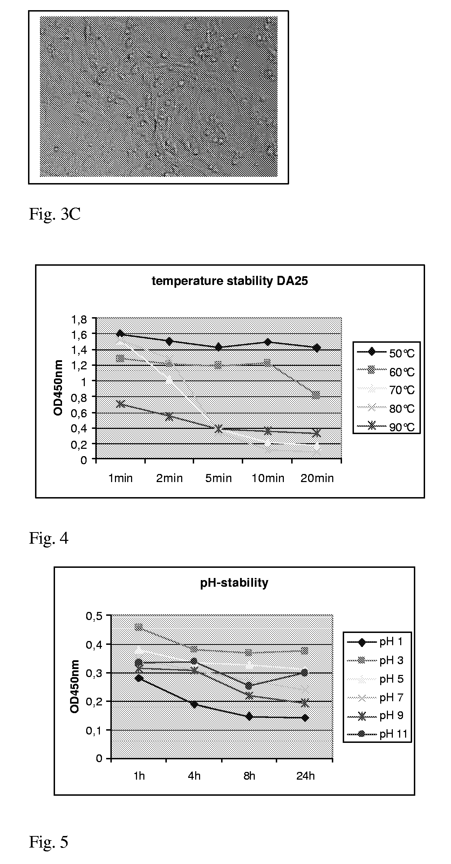

One example of an *in vitro* assay which assesses the neutralizing effect of the antigen-binding polypeptides of the present invention is the "virus neutralization test". Under standard conditions, the virus induces cytopathic changes of the monolayer of susceptible eukaryotic cells (fig. 3B). Antigen-binding polypeptides which specifically bind to bovine coronavirus spike glycoprotein (S2) are added to this system to measure their neutralizing effect on the pathogenic changes (fig. 3C).

Vero cells (African Green Monkey) were grown in MEM medium at 37°C with 5% CO₂ (fig. 3A). Antibody samples were prepared as quadruplicates in serial twofold dilutions at 50 µl/well on 96-well cell culture plates (Nunc). The BCV-V270 stock was diluted in Dulbecco's modified minimal essential medium to 100 50% cell culture infective doses and added at 50 µl/well. The plates were incubated at 37°C in a 5% CO₂ incubator and examined daily with an inverted microscope for BCV-characteristic cytopathic changes, extensive cell fusion, for 4 to 5 days.

The strongest neutralization effect was shown by the scFv antibodies DA10, DA30, DA31 and DA43. None of the negative controls or antibodies against other pathogens showed an inhibitory effect.

**Table 10: ScFv antibodies tested in the virus neutralization test. BB= antibodies against F5-fimbriae, BA= antibodies against F4-fimbriae.**

| scFv | neutralization | | controls | neutralization |
|---|---|---|---|---|
| DA6 | Medium | | no scFv | none |
| DA43 | Strong | | boiled DA43 | none |
| DA30 | Strong | | boiled DA25 | none |
| DA31 | Strong | | BA11 | none |
| DA10 | Strong | | BB 15 | none |
| DA11 | Medium | | | |
| DA25 | Medium | | | |

### Example 4: Stability studies

In order to examine the pH and temperature stability of the antibody fragments, samples were incubated for several time points at different pH and temperatures. For temperature stability 100ng of DA25 antibody was heated to 50°C-90°C and afterwards samples were analyzed by ELISA to quantify the remaining antibody fragments (fig. 4). For pH stability analysis 10µl (250ng) DA25 antibody was incubated with 90µl pH solution (pH 1, 3, 5, 7, 9, 11) and afterwards samples were also analyzed by ELISA to quantify the remaining antibody fragments (fig. 5).

### Example 5: Transient antibody fragment expression in plants

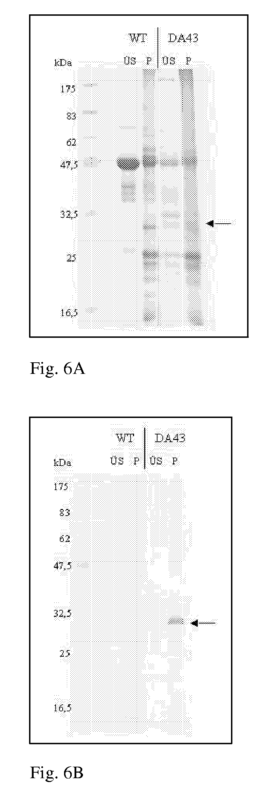

*Nicotiana benthamiana* plants were infected with Agrobacterium (MagnIcon® - System, IconGenetics GmbH, Halle, Germany). Leaf samples were grinded in sample loading buffer and proteins separated on a 15% SDS gel. The proteins were visualized by Coomassie staining (fig. 6A). A second gel was blotted on a nitrocellulose membrane. The membrane was blocked one hour at room temperature (RT) in Rotiblock and afterwards incubated with the primary anti-His antibody (Sigma H1029) diluted 1:3000 in 1 % BSA-TBS/0,05%Tween for one hour at RT. After washing three times with TBS/0,05%Tween the membrane was incubated with the secondary anti-mouse-AP-conjugated antibody (Sigma A4312) diluted 1:30000 in 1 % BSA-TBS/0,05%Tween for one hour at RT. After washing three times with TBS/0,05%Tween and two times five min with TBS the membrane was developed with MBT/BCIP (fig. 6B).

### DESCRIPTION OF THE FIGURES

- Fig. 1A:: Purified spike glycoprotein (S2) expressed in *E.coli* separated on a 15% SDS gel. M = marker; DF = flow through; W = wash; E = eluate.
- Fig. 1B:: Purified spike glycoprotein (S2) expressed in *E.coli* analyzed by Western Blot. M = marker; DF = flow through; W = wash; E = eluate.
- Fig. 2:: scFv antibody DA25 expressed in *E.coli* analyzed by Western Blot.
- Fig. 3A:: Vero Cells grown in MEM medium at 37°C with 5% CO₂.
- Fig. 3B:: Vero Cells incubated with virus (BCV270).
- Fig. 3C:: Vero Cells incubated with virus (BCV270) and 5µg scFv antibody DA43 after 5 days.
- Fig. 4:: Temperature stability of DA25.
- Fig. 5:: pH stability of DA25.
- Fig. 6A:: Leaf crude extracts containing the scFv antibody DA43 separated on a 15% SDS gel. ÜS = supernatant; P = pellet.
- Fig. 6B:: Leaf crude extracts containing the scFv antibody DA43 analyzed by Western Blot. ÜS = supernatant; P = pellet.

## Claims

1. An antigen-binding polypeptide which specifically binds to bovine coronavirus spike glycoprotein (S2).

2. The antigen-binding polypeptide of claim 1, which specifically binds to a polypeptide comprising the amino acid sequence of SEQ ID NO: 1.

3. An antigen-binding polypeptide of claims 1 or 2, comprising:
a first polypeptide comprising the antigen-binding portion of the variable region of an antibody heavy chain, and
a second polypeptide comprising the antigen-binding portion of the variable region of an antibody light chain.

4. The antigen-binding polypeptide of claim 3, which further comprises a peptide linker linking said first and said second polypeptides into a single-chain polypeptide.

5. An antigen-binding polypeptide of claims 3 or 4, wherein said variable region of an antibody heavy chain comprises a VH CDR1, wherein the VH CDR1 has the amino acid sequence depicted in SEQ ID NO: 19.

6. The antigen-binding polypeptide of claim 5, wherein the VH CDR1 has the amino acid sequence depicted in SEQ ID NOs: 16, 17 or 18.

7. An antigen-binding polypeptide of any one of claims 3 to 6, wherein said variable region of an antibody heavy chain comprises a VH CDR2, wherein the VH CDR2 has the amino acid sequence depicted in SEQ ID NO: 27.

8. The antigen-binding polypeptide of claim 7, wherein the VH CDR2 has the amino acid sequence depicted in SEQ ID NOs: 24, 28 or 30.

9. An antigen-binding polypeptide of any one of claims 3 to 8, wherein said variable region of an antibody heavy chain comprises a VH CDR3, wherein the VH CDR3 has the amino acid sequence depicted in SEQ ID NO: 37.

10. The antigen-binding polypeptide of claim 9, wherein the VH CDR3 has the amino acid sequence depicted in SEQ ID NOs: 35, 36 or 38.

11. An antigen-binding polypeptide of any one of claims 3 to 10, wherein said variable region of an antibody light chain comprises a VL CDR1, wherein the VL CDR1 has the amino acid sequence depicted in SEQ ID NO: 45.

12. The antigen-binding polypeptide of claim 11, wherein the VL CDR1 has the amino acid sequence depicted in SEQ ID NOs: 41, 44 or 47.

13. An antigen-binding polypeptide of any one of claims 3 to 12, wherein said variable region of an antibody light chain comprises a VL CDR2, wherein the VL CDR2 has the amino acid sequence depicted in SEQ ID NO: 54.

14. The antigen-binding polypeptide of claim 13, wherein the VL CDR2 has the amino acid sequence depicted in SEQ ID NOs: 52, 53 or 55.

15. An antigen-binding polypeptide of any one of claims 3 to 14, wherein said variable region of an antibody light chain comprises a VL CDR3, wherein the VL CDR3 has the amino acid sequence depicted in SEQ ID NO: 65.

16. The antigen-binding polypeptide of claim 15, wherein the VL CDR3 has the amino acid sequence depicted in SEQ ID NOs: 66, 68 or 69.

17. The single-chain antigen-binding polypeptide of claim 4 comprising the amino acid sequence of SEQ ID NOs: 2, 3, 4, 5, 6, 7 or 8.

18. An antigen-binding polypeptide of any one of claims 4 to 16, as far as they relate to a single-chain polypeptide, wherein said peptide linker is not susceptible to protease cleavage.

19. An antigen-binding polypeptide of any one of claims 4 to 16, as far as they relate to a single-chain polypeptide, wherein said peptide linker has an amino acid sequence of SEQ ID NO:135.

20. An antigen-binding polypeptide of any one of claims 4 to 16, as far as they relate to a single-chain polypeptide, wherein said peptide linker is selected from the group consisting of a GS-linker, a yol-linker or a linker with the amino acid sequence of SEQ ID NO: 134.

21. A multivalent single-chain antigen-binding polypeptide, comprising two or more single-chain polypeptides of any one of claims 4 to 20, wherein the single-chain polypeptides are linked by a peptide linker.

22. The multivalent single-chain antigen-binding polypeptide of claim 20, wherein the peptide linker is a GS-linker.

23. An antigen-binding polypeptide of any one of claims 1 to 3 or 5 to 16, which is a Fab antibody fragment.

24. An antigen-binding polypeptide of claims 1 or 2, which is a non-immunoglobulin polypeptide.

25. A polynucleotide comprising a nucleotide sequence which encodes an antigen-binding polypeptide of any one of the preceding claims.

26. The polynucleotide of claim 25 comprising the nucleotide sequence of SEQ ID NOs: 9, 10, 11, 12, 13, 14 or 15.

27. A vector comprising the polynucleotide of claims 25 or 26.

28. The vector of claim 27 which is a phage display vector, a bacterial expression vector, a yeast expression vector, a fungus expression vector, an algae expression vector or a plant expression vector.

29. The plant expression vector of claim 28 comprising a seed specific promoter.

30. The plant expression vector of claim 29 wherein the promoter is the short or the long USB promoter.

31. A host cell comprising a polynucleotide of claim 25 or 26 or a vector of any one of claims 27 to 30.

32. The host cell of claim 31, which is a bacterial cell, a yeast cell, a fungus cell, an algae cell or a plant cell.

33. The host cell of claim 32, which is an *Escherichia coli* cell, a Streptomyces cell, a *Pichia pastoris* cell, a Schizosaccharomyces cell, an Aspergillus cell, a Chlamydomonas cell, a *Nicotiana spec.* cell, a *Pisum sativum* cell, a *Hordeum vulgare* cell, a *Zea mays* cell, a rapeseed cell, a *Cucumis sativus* cell or a *Linum usitatissimum* cell.

34. Transgenic plants or parts thereof or microorganisms containing a polynucleotide of claim 25 or 26 or a vector of any one of claims 27 to 30.

35. An antigen-binding polypeptide of any one of claims 1 to 24, which neutralizes the cytopathic changes of the cell monolayer caused by bovine coronavirus.

36. An antigen-binding polypeptide of any one of claims 1 to 24, which has a protective and/or a curative effect on infection of mammals with bovine coronavirus .

37. A composition comprising the antigen-binding polypeptide of any one of claims 1 to 24, 35 and 36 and an acceptable excipient, carrier, buffer and/or stabilizer.

38. The composition of claim 37, wherein the excipient, carrier, buffer and/or stabilizer comprises a protein source, preferably BSA, milk powder and/or pea flour; micro-particles and/or gelatine capsules, which are preferably encased with a stabilizing liquid.

39. Use of an antigen-binding polypeptide of any one of claims 1 to 24, 35 and 36 for the manufacture of a medicament for the treatment and/or prevention of infections with bovine coronavirus.

40. Use of an antigen-binding polypeptide of any one of claims 1 to 24, 35 and 36 for detecting bovine coronavirus spike glycoprotein (S2).

41. Use of the plants, plant parts or microorganisms of claim 32 as fodder for mammals.

42. Use according to claim 41, wherein the mammals are cattle.

43. A method of producing an antigen-binding polypeptide of any one of claims 1 to 24, 35 and 36, comprising:
providing an expression vector of any one of claims 27 to 30, and expressing said vector in a host cell.


SEQUENCE LISTING

<110>  Novoplant GmbH

<120>  Antigen binding polypeptides against spike glycoprotein (S2) of
       bovine coronavirus

<130>  N7353/RN

<150>  EP 06 114 237.8
<151>  2006-05-19

<160>  148

<170>  PatentIn version 3.3

<210>  1
<211>  214
<212>  PRT
<213>  Bovine coronavirus

<400>  1

Val Asn Glu Cys Val Lys Ser Gln Ser Ser Arg Ile Asn Phe Cys Gly
1               5                   10                  15

Asn Gly Asn His Ile Ile Ser Leu Val Gln Asn Ala Pro Tyr Gly Leu
            20                  25                  30

Tyr Phe Ile His Phe Ser Tyr Val Pro Thr Lys Tyr Val Thr Ala Lys
        35                  40                  45

Val Ser Pro Gly Leu Cys Ile Ala Gly Asp Arg Gly Ile Ala Pro Lys
    50                  55                  60

Ser Gly Tyr Phe Val Asn Val Asn Asn Thr Trp Met Phe Thr Gly Ser
65                  70                  75                  80

Gly Tyr Tyr Tyr Pro Glu Pro Ile Thr Gly Asn Asn Val Val Val Met
                85                  90                  95

Ser Thr Cys Ala Val Asn Tyr Thr Lys Ala Pro Asp Val Met Leu Asn
            100                 105                 110

Ile Ser Thr Pro Asn Leu Pro Asp Phe Lys Glu Glu Leu Asp Gln Trp
        115                 120                 125

Phe Lys Asn Gln Thr Ser Val Ala Pro Asp Leu Ser Leu Asp Tyr Ile
    130                 135                 140

Asn Val Thr Phe Leu Asp Leu Gln Asp Glu Met Asn Arg Leu Gln Glu
145                 150                 155                 160

Ala Ile Lys Val Leu Asn Gln Ser Tyr Ile Asn Leu Lys Asp Ile Gly
                165                 170                 175

Thr Tyr Glu Tyr Tyr Val Lys Trp Pro Trp Tyr Val Trp Leu Leu Ile

180                 185                 190

Gly Leu Ala Gly Val Ala Met Leu Val Leu Leu Phe Phe Ile Cys Cys
        195                 200                 205

Cys Thr Gly Cys Gly Thr
    210

<210>  2
<211>  281
<212>  PRT
<213>  Artificial

<220>
<223>  Polypeptide DA6

<400>  2

Met Ala Glu Val Gln Leu Gln Gln Ser Gly Ala Glu Leu Val Arg Pro
1               5                   10                  15

Gly Thr Ser Val Lys Val Ser Cys Lys Ala Ser Gly Tyr Ala Phe Thr
            20                  25                  30

Asn Tyr Leu Ile Glu Trp Val Lys Lys Arg Pro Gly Gln Gly Leu Glu
        35                  40                  45

Trp Ile Gly Val Ile Asn Pro Gly Ser Gly Gly Thr Asn Tyr Asn Glu
    50                  55                  60

Lys Phe Lys Gly Lys Ala Thr Leu Thr Ala Asp Lys Ser Ser Ser Thr
65                  70                  75                  80

Ala His Met Gln Leu Ser Ser Leu Thr Ser Asp Asp Ser Ala Val Tyr
                85                  90                  95

Phe Cys Ala Arg Tyr Thr Leu Tyr Tyr Gly Asn Pro Tyr Tyr Tyr Ala
            100                 105                 110

Met Asp Tyr Trp Gly Gln Gly Thr Ser Val Thr Val Ser Ser Ala Lys
        115                 120                 125

Thr Thr Pro Pro Ser Val Lys Leu Glu Glu Gly Glu Phe Ser Glu Ala
    130                 135                 140

Arg Val Gln Ala Val Val Thr Gln Glu Ser Ala Leu Thr Thr Ser Pro
145                 150                 155                 160

Gly Glu Thr Val Thr Leu Thr Cys Arg Ser Ser Thr Gly Ala Val Thr
                165                 170                 175

Thr Ser Asn Tyr Ala Asn Trp Val Gln Glu Lys Pro Asp His Leu Phe
            180                 185                 190

Thr Gly Leu Ile Gly Gly Thr Asn Asn Arg Pro Pro Gly Val Pro Ala
        195                 200                 205

Arg Phe Ser Gly Ser Leu Ile Gly Asp Lys Ala Ala Leu Thr Ile Thr
    210                 215                 220

Gly Ala Gln Thr Glu Asp Glu Ala Ile Tyr Phe Cys Ala Leu Trp Tyr
225                 230                 235                 240

Ser Asn His Trp Val Phe Gly Gly Gly Thr Lys Leu Thr Val Leu Gly
                245                 250                 255

Gln Pro Lys Ser Ser Pro Ser Val Thr Leu Phe Pro Pro Ser Ala Ala
            260                 265                 270

Ala Gly Ser His His His His His His
        275                 280

<210>  3
<211>  265
<212>  PRT
<213>  Artificial

<220>
<223>  Polypeptide DA10

<400>  3

Met Ala Glu Val Gln Leu Gln Gln Ser Gly Ala Glu Leu Val Lys Pro
1               5                   10                  15

Gly Ala Ser Val Lys Leu Ser Cys Thr Ala Ser Gly Phe Asn Ile Lys
            20                  25                  30

Asp Thr Tyr Ile His Trp Val Lys Gln Arg Pro Glu Gln Gly Leu Glu
        35                  40                  45

Trp Ile Gly Arg Ile Asp Pro Ala Ile Gly Asn Thr Lys Tyr Asp Pro
    50                  55                  60

Lys Phe Gln Gly Lys Ala Thr Ile Thr Ala Asp Thr Ser Ser Asn Thr
65                  70                  75                  80

Ala Tyr Leu His Leu Ser Ser Leu Thr Ser Glu Asp Thr Ala Val Tyr
                85                  90                  95

Tyr Cys Ala Arg Arg Asn Phe Tyr Ala Met Asp Tyr Trp Gly Gln Gly
            100                 105                 110

Thr Ser Val Thr Val Ser Ser Ala Lys Thr Thr Ala Pro Ser Val Lys
        115                 120                 125

Leu Glu Glu Gly Glu Phe Ser Glu Ala Arg Val Asp Ile Gln Met Thr

130                 135                 140

Gln Thr Thr Ser Ser Leu Ser Ala Ser Leu Gly Asp Arg Val Thr Ile
145                 150                 155                 160

Ser Cys Ser Ala Ser Gln Gly Ile Ser Asn Tyr Leu Asn Trp Tyr Gln
                165                 170                 175

Gln Lys Pro Asp Gly Thr Val Lys Leu Leu Ile Tyr Tyr Thr Ser Ser
            180                 185                 190

Leu His Ser Gly Val Pro Ser Arg Phe Ser Gly Ser Gly Ser Gly Thr
        195                 200                 205

Asp Tyr Ser Leu Thr Ile Ser Asn Leu Glu Pro Glu Asp Ile Ala Thr
    210                 215                 220

Tyr Tyr Cys Gln Gln Tyr Ser Lys Leu Pro Arg Thr Phe Gly Gly Gly
225                 230                 235                 240

Thr Lys Leu Glu Ile Lys Arg Ala Asp Ala Ala Pro Thr Val Ala Ala
                245                 250                 255

Ala Gly Ser His His His His His His
            260                 265

<210>  4
<211>  264
<212>  PRT
<213>  Artificial

<220>
<223>  Polypeptide DA11

<400>  4

Met Ala Glu Val Lys Leu Val Glu Ser Gly Gly Gly Leu Val Lys Pro
1               5                   10                  15

Gly Gly Ser Leu Lys Val Ser Cys Ala Ala Ser Gly Phe Thr Phe Ser
            20                  25                  30

Asn Tyr Ala Met Ser Trp Val Arg Gln Thr Pro Glu Lys Arg Leu Glu
        35                  40                  45

Trp Val Ala Ser Ile Asn Ser Gly Gly Tyr Thr Tyr Tyr Pro Asp Ser
    50                  55                  60

Val Lys Gly Arg Phe Thr Ile Ser Arg Asp Asn Ala Arg Asn Ile Leu
65                  70                  75                  80

Tyr Leu Gln Met Ser Ser Leu Arg Ser Glu Asp Thr Ala Ile Tyr Tyr
                85                  90                  95

Cys Ala Arg Gly Gly Arg Pro Leu Tyr Phe Asp Tyr Trp Gly Gln Gly
            100                 105                 110

Thr Thr Leu Thr Val Ser Ser Ala Lys Thr Thr Pro Pro Ser Val Lys
        115                 120                 125

Leu Glu Glu Gly Glu Phe Ser Glu Ala Arg Val Gln Ile Val Leu Thr
    130                 135                 140

Gln Ser Pro Ala Ile Met Ser Ala Ser Pro Gly Glu Lys Val Thr Met
145                 150                 155                 160

Thr Cys Ser Ala Ser Ser Ser Val Ser Tyr Met His Trp Tyr Gln Gln
                165                 170                 175

Lys Ser Gly Thr Ser Pro Lys Arg Trp Ile Tyr Asp Thr Ser Lys Leu
            180                 185                 190

Ala Ser Gly Val Pro Gly Arg Phe Ser Gly Ser Gly Ser Gly Thr Ser
        195                 200                 205

Tyr Ser Leu Thr Ile Ser Ser Met Glu Ala Glu Asp Ala Ala Thr Tyr
    210                 215                 220

Tyr Cys Gln Gln Trp Ser Ser Asn Pro Pro Thr Phe Gly Ser Gly Thr
225                 230                 235                 240

Lys Leu Glu Ile Lys Arg Ala Asp Ala Ala Pro Thr Val Ala Ala Ala
                245                 250                 255

Gly Ser His His His His His His
            260

<210>  5
<211>  264
<212>  PRT
<213>  Artificial

<220>
<223>  Polypeptide DA25

<400>  5

Met Ala Glu Val Gln Leu Val Glu Ser Gly Gly Gly Leu Val Lys Pro
1               5                   10                  15

Gly Gly Ser Leu Lys Leu Ser Cys Ala Ala Ser Gly Phe Thr Phe Ser
            20                  25                  30

Asn Tyr Ala Met Ser Trp Val Arg Gln Thr Pro Glu Lys Arg Leu Glu
        35                  40                  45

Trp Val Ala Ser Ile Thr Ser Gly Gly Ile Thr Tyr Tyr Pro Asp Ser

50                  55                  60

Val Lys Gly Arg Phe Thr Ile Ser Arg Asp Asn Ala Arg Asn Ile Leu
65                  70                  75                  80

Tyr Leu Gln Met Ser Ser Leu Arg Ser Glu Asp Thr Ala Met Tyr Tyr
                85                  90                  95

Cys Thr Arg Gly Gly Arg Pro Leu Tyr Phe Asp Tyr Trp Gly Gln Gly
            100                 105                 110

Thr Thr Leu Thr Val Ser Ser Ala Lys Thr Thr Pro Pro Ser Val Lys
        115                 120                 125

Leu Glu Glu Gly Glu Phe Ser Glu Ala Arg Val Gln Ile Val Leu Thr
    130                 135                 140

Gln Ser Pro Ala Ile Met Ser Ala Ser Pro Gly Glu Lys Val Thr Met
145                 150                 155                 160

Thr Cys Ser Ala Ser Ser Ser Val Ser Tyr Met His Trp Tyr Gln Gln
                165                 170                 175

Lys Ser Gly Thr Ser Pro Lys Arg Trp Val Tyr Asp Thr Ser Lys Leu
            180                 185                 190

Ala Ser Gly Val Pro Ser Arg Phe Ser Gly Ser Gly Ser Gly Thr Ser
        195                 200                 205

Tyr Ser Leu Thr Ile Ser Ser Met Glu Ala Glu Asp Ala Ala Thr Tyr
    210                 215                 220

Tyr Cys Gln Gln Trp Ser Ser Asn Pro Leu Thr Phe Gly Ala Gly Thr
225                 230                 235                 240

Lys Leu Glu Leu Lys Arg Ala Asp Ala Ala Pro Thr Val Ala Ala Ala
                245                 250                 255

Gly Ser His His His His His His
            260

<210>  6
<211>  281
<212>  PRT
<213>  Artificial

<220>
<223>  Polypeptide DA30

<400>  6

Met Ala Gln Val Gln Leu Gln Gln Ser Gly Ala Glu Leu Val Arg Pro
1               5                   10                  15

Gly Thr Ser Val Lys Val Ser Cys Lys Ala Ser Gly Tyr Ala Phe Thr
            20                  25                  30

Asn Tyr Leu Ile Glu Trp Val Lys Gln Arg Pro Gly Gln Gly Leu Glu
        35                  40                  45

Trp Ile Gly Val Ile Asn Pro Gly Ser Gly Gly Thr Asn Tyr Asn Glu
    50                  55                  60

Lys Phe Lys Asp Lys Ala Thr Leu Thr Ala Asp Lys Ser Ser Ser Thr
65                  70                  75                  80

Ala Tyr Met Gln Leu Ser Ser Leu Thr Ser Asp Asp Ser Ala Val Tyr
                85                  90                  95

Phe Cys Ala Arg Phe Thr Leu Tyr His Gly Asn Pro Tyr Tyr Tyr Ala
            100                 105                 110

Met Asp Tyr Trp Gly Gln Gly Thr Ser Val Thr Val Ser Ser Ala Lys
        115                 120                 125

Thr Thr Ala Pro Ser Val Lys Leu Glu Glu Gly Glu Phe Ser Glu Ala
    130                 135                 140

Arg Val Gln Ala Val Val Thr Gln Glu Ser Ala Leu Thr Thr Ser Pro
145                 150                 155                 160

Gly Glu Thr Val Thr Leu Thr Cys Arg Ser Ser Thr Gly Ala Val Thr
                165                 170                 175

Thr Ser Asn Tyr Ala Asn Trp Val Gln Glu Lys Pro Asp His Leu Phe
            180                 185                 190

Thr Gly Leu Ile Gly Gly Thr Asn Asn Arg Ala Pro Gly Val Pro Ala
        195                 200                 205

Arg Phe Ser Gly Ser Leu Ile Gly Asp Lys Ala Ala Leu Thr Ile Thr
    210                 215                 220

Gly Ala Gln Thr Glu Asp Glu Ala Ile Tyr Phe Cys Ala Leu Trp Tyr
225                 230                 235                 240

Ser Asn His Trp Val Phe Gly Gly Gly Thr Lys Leu Thr Val Leu Gly
                245                 250                 255

Gln Pro Lys Ser Ser Pro Ser Val Thr Leu Phe Pro Pro Ser Ala Ala
            260                 265                 270

Ala Gly Ser His His His His His His
        275                 280

<210>  7
<211>  272
<212>  PRT
<213>  Artificial

<220>
<223>  Polypeptide DA31

<400>  7

Met Ala Glu Val Gln Leu Gln Gln Ser Gly Ala Glu Leu Val Arg Pro
1               5                   10                  15

Gly Thr Ser Val Lys Val Ser Cys Lys Ala Ser Gly Tyr Ala Phe Thr
            20                  25                  30

Asn Tyr Leu Ile Glu Trp Val Lys Gln Arg Pro Gly Gln Gly Leu Glu
        35                  40                  45

Trp Ile Gly Val Ile Asn Pro Gly Ser Gly Gly Thr Asn Tyr Asn Glu
    50                  55                  60

Lys Phe Lys Gly Lys Ala Thr Leu Thr Ala Asp Lys Ser Ser Ser Thr
65                  70                  75                  80

Ala Tyr Met Gln Leu Ser Ser Leu Thr Ser Asp Asp Ser Ala Val Tyr
                85                  90                  95

Phe Cys Ala Arg Tyr Thr Leu Tyr Tyr Gly Asn Pro Tyr Tyr Tyr Ala
            100                 105                 110

Met Glu Tyr Trp Gly Gln Gly Thr Ser Val Thr Val Ser Ser Ala Lys
        115                 120                 125

Thr Thr Pro Pro Ser Val Lys Leu Glu Glu Gly Glu Phe Ser Glu Ala
    130                 135                 140

Arg Val Asp Ile Gln Met Thr Gln Thr Thr Ser Ser Leu Ser Ala Ser
145                 150                 155                 160

Leu Gly Asp Arg Val Thr Ile Ser Cys Arg Ala Ser Gln Asp Ile Ser
                165                 170                 175

Asn Tyr Leu Asn Trp Tyr Gln Gln Lys Pro Asp Gly Thr Val Lys Leu
            180                 185                 190

Leu Ile Tyr Tyr Thr Ser Arg Leu His Ser Gly Val Pro Ser Arg Phe
        195                 200                 205

Ser Gly Ser Gly Ser Gly Thr Asp Tyr Ser Leu Thr Ile Ser Asn Leu
    210                 215                 220

Glu Gln Glu Asp Ile Ala Thr Tyr Phe Cys Gln Gln Gly Asn Thr Leu

225                 230                 235                 240

Pro Pro Thr Phe Gly Gly Gly Thr Lys Leu Glu Ile Lys Arg Pro Asp
                245                 250                 255

Ala Ala Pro Thr Val Ala Ala Ala Gly Ser His His His His His His
            260                 265                 270

<210>  8
<211>  281
<212>  PRT
<213>  Artificial

<220>
<223>  Polypeptide DA43

<400>  8

Met Ala Glu Val Gln Leu Gln Gln Ser Gly Ala Glu Leu Val Arg Pro
1               5                   10                  15

Gly Thr Ser Val Lys Val Ser Cys Lys Ala Ser Gly Tyr Ala Phe Thr
            20                  25                  30

Asn Tyr Leu Ile Glu Trp Val Lys Gln Arg Pro Gly Gln Gly Leu Glu
        35                  40                  45

Trp Ile Gly Val Ile Asn Pro Gly Ser Gly Gly Thr Asn Tyr Asn Glu
    50                  55                  60

Lys Phe Lys Gly Lys Ala Thr Leu Thr Ala Asp Lys Ser Ser Ser Thr
65                  70                  75                  80

Ala Tyr Met Gln Leu Ser Ser Leu Thr Ser Asp Asp Ser Ala Val Tyr
                85                  90                  95

Phe Cys Ala Arg Tyr Thr Leu Tyr Tyr Gly Asn Pro Tyr Tyr Tyr Ala
            100                 105                 110

Met Asp Tyr Trp Gly Gln Gly Thr Ser Val Thr Val Ser Ser Ala Lys
        115                 120                 125

Thr Thr Pro Pro Ser Val Lys Leu Glu Glu Gly Glu Phe Ser Glu Ala
    130                 135                 140

Arg Val Gln Ala Val Val Thr Gln Glu Ser Ala Leu Thr Thr Ser Pro
145                 150                 155                 160

Gly Glu Thr Val Thr Leu Thr Cys Arg Ser Ser Thr Gly Ala Val Thr
                165                 170                 175

Thr Ser Asn Tyr Ala Asn Trp Val Gln Glu Lys Pro Asp His Leu Phe
            180                 185                 190

Thr Gly Leu Ile Gly Gly Thr Asn Asn Arg Ala Pro Gly Val Pro Ala
        195                 200                 205

Arg Phe Ser Gly Ser Leu Ile Gly Asp Lys Ala Thr Leu Thr Ile Thr
    210                 215                 220

Gly Ala Gln Thr Glu Asp Glu Ala Ile Tyr Phe Cys Ala Leu Trp Phe
225                 230                 235                 240

Ser Asn His Trp Val Phe Gly Gly Gly Thr Lys Leu Thr Val Leu Gly
                245                 250                 255

Gln Pro Lys Ser Ser Pro Ser Val Thr Leu Phe Pro Pro Ser Ala Ala
            260                 265                 270

Ala Gly Ser His His His His His His
        275                 280

<210>  9
<211>  843
<212>  DNA
<213>  Artificial

<220>
<223>  Nucleotide sequence encoding polypeptide DA6

<400>  9
atggcggagg tccagctgca acaatctgga gctgagctgg taaggcctgg gacttcagtg      60

aaggtgtcct gcaaggcttc tggatacgcc ttcactaatt acttgataga gtgggtaaag     120

aagaggcctg gacagggcct tgagtggatt ggagtgatta atcctggaag tggtggtact     180

aactacaatg agaagttcaa gggcaaggca acactgactg cagacaaatc ctccagcact     240

gcccacatgc agctcagcag cctgacatct gatgactctg cggtctattt ctgtgcaaga     300

tataccctct actatggtaa cccttattac tatgctatgg actactgggg tcaaggaacc     360

tcagtcaccg tctcctcagc caaaacaaca cccccatcag tcaagcttga agaaggtgaa     420

ttttcagaag cacgcgtaca ggctgttgtg actcaggaat ctgcactcac cacatcacct     480

ggtgaaacag tcacactcac ttgtcgctca agtactgggg ctgttacaac tagtaactat     540

gccaactggg tccaagaaaa accagatcat ttattcactg gtctaatagg tggtaccaac     600

aaccgacctc caggtgttcc tgccagattc tcaggctccc tgattggaga caaggctgcc     660

ctcaccatca caggggcaca gactgaggat gaggcaatat atttctgtgc tctatggtac     720

agcaaccatt gggtgttcgg tggaggaacc aaactgactg tcctaggcca gcccaagtct     780

tcgccatcag tcaccctgtt tccaccttcc gcggccgctg gatcccatca ccatcaccat     840

cac                                                                   843

<210>  10
<211>  795
<212>  DNA

<213>  Artificial

<220>
<223>  Nucleotide sequence encoding polypeptide DA10

<400>  10
atggcggagg tccagctgca gcagtctggg gcagagcttg tgaagcctgg ggcctcagtc      60

aagttgtcct gcacagcttc tggcttcaac attaaagaca cctatataca ctgggtgaag     120

cagaggcctg aacagggcct ggagtggatt ggaaggattg atcctgcgat tggtaatact     180

aaatatgacc cgaagttcca gggcaaggcc actataacag cggacacatc ctccaacaca     240

gcctacctgc acctcagcag cctgacatct gaggacactg ccgtctatta ctgtgctaga     300

aggaatttct atgctatgga ctactggggt caaggaacct cagtcaccgt ctcctcagcc     360

aaaacaacag ccccatcggt caagcttgaa gaaggtgaat tttcagaagc acgcgtagat     420

atccagatga cacagactac atcctccctg tctgcctctc tgggagacag agtcaccatc     480

agttgcagtg caagtcaggg cattagcaat tatttaaact ggtatcagca gaaaccagat     540

ggaactgtta aactcctgat ctattacaca tcaagtttac actcaggagt cccatcaagg     600

ttcagtggca gtgggtctgg gacagattat tctctcacca tcagcaacct ggaacctgaa     660

gatattgcca cttactattg tcagcagtat agtaagcttc ctcggacgtt cggtggaggc     720

accaagctgg aaatcaaacg ggctgatgct gcaccaactg tagcggccgc tggatcccat     780

caccatcacc atcac                                                      795


<210>  11
<211>  792
<212>  DNA
<213>  Artificial

<220>
<223>  Nucleotide sequence encoding polypeptide DA11


<220>
<221>  misc_feature
<222>  (720)..(720)
<223>  n is a, c, g, or t

<220>
<221>  misc_feature
<222>  (732)..(732)
<223>  n is a, c, g, or t

<400>  11
atggcggagg tgaagctggt ggagtctggg ggaggcttag tgaagcctgg agggtccctg      60

aaagtctcct gtgcagcctc tggattcact ttcagtaact atgccatgtc ttgggttcgc     120

cagactccag agaagaggct ggagtgggtc gcatccatta atagtggtgg ttacacctac     180

tatccagaca gtgtgaaggg ccgattcacc atctccagag ataatgccag gaacattctg     240

tacctgcaaa tgagcagtct gaggtctgag gacacggcca tatattactg tgcaagaggc     300

ggacgacccc tttactttga ctactggggc caaggcacca ctctcacagt ctcctcagcc     360

aaaacaacac ccccatcagt caagcttgaa gaaggtgaat tttcagaagc acgcgtacaa     420

attgttctca cccagtctcc agcaatcatg tctgcatctc caggggagaa ggtcaccatg 480

acctgcagtg ccagctcaag tgtaagttac atgcactggt accagcagaa gtcaggcacc 540

tcccccaaaa gatggattta tgacacatcc aaactggctt ctggagtccc tggtcgcttc 600

agtggcagtg ggtctgggac ctcttactct ctcacaatta gcagcatgga ggctgaagat 660

gctgccactt attactgcca gcagtggagt agtaacccac caacgttcgg ctcggggacn 720

aagttggaaa tnaaacgggc tgatgctgca ccaactgtag cggccgctgg atcccatcac 780

catcaccatc ac 792

<210> 12
<211> 792
<212> DNA
<213> Artificial

<220>
<223> Nucleotide sequence encoding polypeptide DA25

<400> 12
atggcggaag tgcagctggt ggagtctggg ggaggcttag tgaagcctgg agggtccctg 60

aaactctcct gtgcagcctc tggattcact ttcagtaact atgccatgtc ttgggttcgc 120

cagactccag agaagaggct ggagtgggtc gcatccatta ctagtggcgg tattacctac 180

tatccagaca gtgtgaaggg ccgattcacc atctccagag ataatgccag gaacatcctg 240

tacctgcaaa tgagcagtct gaggtctgag gacacggcca tgtattactg tacaagaggc 300

ggacggcccc tttactttga ctactggggc caaggcacca ctctcacggt ctcctcagcc 360

aaaacgacac ccccatctgt caagcttgaa gaaggtgaat tttcagaagc acgcgtacaa 420

attgttctca cccagtctcc agcaatcatg tctgcatctc ctggggagaa ggtcaccatg 480

acctgcagtg ccagctcaag tgtaagttac atgcactggt atcagcagaa gtcaggcacc 540

tcccccaaaa gatgggttta tgacacatcc aaactggctt ctggagtccc ttctcgcttc 600

agtggcagtg ggtctgggac ctcttactct ctcacaatca gcagcatgga ggctgaagat 660

gctgccactt attactgcca gcagtggagt agtaacccgc tcacgttcgg tgctgggacc 720

aagctggagc tgaaacgggc tgatgctgca ccaactgtag cggccgctgg atcccatcac 780

catcaccatc ac 792

<210> 13
<211> 843
<212> DNA
<213> Artificial

<220>
<223> Nucleotide sequence encoding polypeptide DA30

<400> 13
atggcgcagg ttcaactgca gcagtctgga gctgagctgg taaggcctgg gacttcagtg 60

aaggtgtcct gcaaggcttc tggatacgcc ttcactaatt acttgataga gtgggtaaag 120

cagaggcctg gacagggcct tgagtggatt ggagtgatta atcctggaag tggtggtact 180

aactacaatg agaagttcaa ggacaaggca acactgactg cagacaagtc ctccagcact 240

gcctacatgc agctcagcag cctgacatct gatgactctg cggtctattt ctgtgcaaga 300

tttaccctct atcatggtaa cccttattac tatgctatgg actactgggg tcaaggaacc 360

tcagtcaccg tctcctcagc caaaacaaca gccccatcgg tcaagcttga agaaggtgaa 420

ttttcagaag cacgcgtaca ggctgttgtg actcaggaat ctgcactcac cacatcacct 480

ggtgaaacag tcacactcac ttgtcgctca agtactgggg ctgttacaac tagtaactat 540

gccaactggg tccaagaaaa accagatcat ttattcactg gtctaatagg tggtaccaac 600

aaccgagctc caggtgttcc tgccagattc tcaggctccc tgattggaga caaggctgcc 660

ctcaccatca caggggcaca gactgaggat gaggcaatat atttctgtgc tctatggtac 720

agcaaccact gggtgttcgg tggaggaacc aaactgactg tcctaggcca gcccaagtct 780

tcgccatcag tcaccctgtt tccaccttcc gcggccgctg gatcccatca ccatcaccat 840

cac 843

<210> 14
<211> 816
<212> DNA
<213> Artificial

<220>
<223> Nucleotide sequence encoding polypeptide DA31

<400> 14
atggcggagg tccagctgca acaatctgga gctgagctgg taaggcctgg gacttcagtg 60

aaggtgtcct gcaaggcttc tggatacgcc ttcactaatt acttgataga gtgggtaaag 120

cagaggcctg gacagggcct tgagtggatt ggagtgatta atcctggaag tggtggtact 180

aactacaatg agaagttcaa gggcaaggca acactgactg cagacaaatc ctccagcact 240

gcctacatgc agctcagcag cctgacatct gatgactctg cggtctattt ctgtgcaaga 300

tataccctct actatggtaa cccttattac tatgctatgg agtactgggg tcaaggaacc 360

tcagtcaccg tctcctcagc caaaacgaca cccccatctg tcaagcttga agaaggtgaa 420

ttttcagaag cacgcgtaga tatccagatg acacagacta catcctccct gtctgcctct 480

ctgggagaca gagtcaccat cagttgcagg gcaagtcagg acattagcaa ttatttaaac 540

tggtatcagc agaaaccaga tggaactgtt aaactcctga tctactacac atcaagatta 600

cactcaggag tcccatcaag gttcagtggc agtgggtctg gaacagatta ttctctcacc 660

attagcaacc tggagcaaga agatattgcc acttacttct gccaacaggg taatacgctt 720

cctccgacgt tcggtggagg caccaagctg gaaatcaaac ggcctgatgc tgcaccaact 780

gtagcggccg ctggatccca tcaccatcac catcac 816

<210> 15
<211> 843
<212> DNA
<213> Artificial

<220>
<223>  Nucleotide sequence encoding polypeptide DA43

<400>  15
atggcggagg tccagctgca acagtctgga gctgagctgg taaggcctgg gacttcagtg      60

aaggtgtcct gcaaggcttc tggatacgcc ttcactaatt acttgataga gtgggtaaag     120

cagaggcctg gacagggcct tgagtggatt ggagtgatta atcctggaag tggtggtact     180

aactacaatg agaagttcaa gggcaaggca acactgactg cagacaaatc ctccagcact     240

gcctacatgc agctcagcag cctgacatct gatgactctg cggtctattt ctgtgcaaga     300

tataccctct actatggtaa cccttattac tatgctatgg actactgggg tcaaggaacc     360

tcagtcaccg tctcctcagc caaaacaaca cccccatcag tcaagcttga agaaggtgaa     420

ttttcagaag cacgcgtaca ggctgttgtg actcaggaat ctgcactcac cacatcacct     480

ggtgaaacag tcacactcac ttgtcgctca agtactgggg ctgttacaac tagtaactat     540

gccaactggg tccaagaaaa accagatcat ttattcactg gtctaatagg tggtaccaac     600

aaccgagctc caggtgttcc tgccagattc tcaggctccc tgattggaga caaggctacc     660

ctcaccatca caggggcaca gactgaggat gaggcaatat atttctgtgc tctatggttc     720

agcaaccatt gggtgttcgg tggaggaacc aaactgactg tcctaggcca gcccaagtct     780

tcgccatcag tcaccctgtt tccaccttcc gcggccgctg gatcccatca ccatcaccat     840

cac                                                                   843

<210>  16
<211>  5
<212>  PRT
<213>  Artificial

<220>
<223>  VH CDR1

<400>  16

Asn Tyr Leu Ile Glu
1               5

<210>  17
<211>  5
<212>  PRT
<213>  Artificial

<220>
<223>  VH CDR1

<400>  17

Asp Thr Tyr Ile His
1               5

<210>  18
<211>  5
<212>  PRT
<213>  Artificial

<220>
<223>  VH CDR1

<400>  18

Asn Tyr Ala Met Ser
1               5


<210>  19
<211>  5
<212>  PRT
<213>  Artificial

<220>
<223>  VH CDR1


<220>
<221>  misc_feature
<222>  (1)..(5)
<223>  Xaa can be any naturally occurring amino acid

<400>  19

Xaa Xaa Xaa Xaa Xaa
1               5


<210>  20
<211>  5
<212>  PRT
<213>  Artificial

<220>
<223>  VH CRD1


<220>
<221>  misc_feature
<222>  (1)..(3)
<223>  Xaa can be any naturally occurring amino acid

<220>
<221>  misc_feature
<222>  (5)..(5)
<223>  Xaa can be any naturally occurring amino acid

<400>  20

Xaa Xaa Xaa Ile Xaa
1               5


<210>  21
<211>  5
<212>  PRT
<213>  Artificial

<220>
<223>  VH CDR1


<220>
<221>  misc_feature
<222>  (3)..(5)
<223>  Xaa can be any naturally occurring amino acid

<400>  21

Asn Tyr Xaa Xaa Xaa
1               5


<210>  22
<211>  17
<212>  PRT
<213>  Artificial

<220>
<223>  VH CDR2

<400>  22

Val Ile Asn Pro Gly Ser Gly Gly Thr Asn Tyr Asn Glu Lys Phe Lys
1               5                   10                  15


Gly


<210>  23
<211>  17
<212>  PRT
<213>  Artificial

<220>
<223>  VH CDR2

<400>  23

Val Ile Asn Pro Gly Ser Gly Gly Thr Asn Tyr Asn Glu Lys Phe Lys
1               5                   10                  15


Asp


<210>  24
<211>  17
<212>  PRT
<213>  Artificial

<220>
<223>  VH CDR2

<400>  24

Arg Ile Asp Pro Ala Ile Gly Asn Thr Lys Tyr Asp Pro Lys Phe Gln
1               5                   10                  15


Gly


<210>  25
<211>  16
<212>  PRT
<213>  Artificial

<220>
<223>  VH CDR2

<400>  25

Ser Ile Asn Ser Gly Gly Tyr Thr Tyr Tyr Pro Asp Ser Val Lys Gly
1               5                   10                  15


<210>  26
<211>  16
<212>  PRT
<213>  Artificial

<220>
<223>  VH CDR2

<400>  26

Ser Ile Thr Ser Gly Gly Ile Thr Tyr Tyr Pro Asp Ser Val Lys Gly
1               5                   10                  15


<210>  27
<211>  17
<212>  PRT
<213>  Artificial

<220>
<223>  VH CDR2


<220>
<221>  misc_feature
<222>  (1)..(1)
<223>  Xaa can be any naturally occurring amino acid

<220>
<221>  misc_feature
<222>  (3)..(6)
<223>  Xaa can be any naturally occurring amino acid

<220>
<221>  misc_feature
<222>  (8)..(8)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (12)..(17)
<223>  Xaa can be any naturally occurring amino acid

<400>  27

Xaa Ile Xaa Xaa Xaa Xaa Gly Xaa Thr Xaa Tyr Xaa Xaa Xaa Xaa Xaa
1               5                   10                  15


Xaa



<210>  28
<211>  17
<212>  PRT

<213>  Artificial

<220>
<223>  VH CDR2


<220>
<221>  misc_feature
<222>  (17)..(17)
<223>  Xaa can be any naturally occurring amino acid

<400>  28

Val Ile Asn Pro Gly Ser Gly Gly Thr Asn Tyr Asn Glu Lys Phe Lys
1               5                   10                  15


Xaa




<210>  29
<211>  17
<212>  PRT
<213>  Artificial

<220>
<223>  VH CRD2


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (8)..(8)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (12)..(13)
<223>  Xaa can be any naturally occurring amino acid

<220>
<221>  misc_feature
<222>  (16)..(17)
<223>  Xaa can be any naturally occurring amino acid

<400>  29

Xaa Ile Xaa Pro Xaa Xaa Gly Xaa Thr Xaa Tyr Xaa Xaa Lys Phe Xaa

1               5                   10                  15

Xaa

<210>  30
<211>  16
<212>  PRT
<213>  Artificial

<220>
<223>  VH CDR2

<220>
<221>  misc_feature
<222>  (3)..(3)
<223>  Xaa can be any naturally occurring amino acid

<220>
<221>  misc_feature
<222>  (7)..(7)
<223>  Xaa can be any naturally occurring amino acid

<400>  30

Ser Ile Xaa Ser Gly Gly Xaa Thr Tyr Tyr Pro Asp Ser Val Lys Gly
1               5                   10                  15

<210>  31
<211>  17
<212>  PRT
<213>  Artificial

<220>
<223>  VH CDR2

<220>
<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<220>
<221>  misc_feature
<222>  (8)..(8)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (12)..(15)
<223>  Xaa can be any naturally occurring amino acid

<220>
<221>  misc_feature

<222>  (17)..(17)
<223>  Xaa can be any naturally occurring amino acid

<400>  31

Xaa Ile Xaa Xaa Gly Ser Gly Xaa Thr Xaa Tyr Xaa Xaa Xaa Xaa Lys
1               5                   10                  15

Xaa

<210>  32
<211>  15
<212>  PRT
<213>  Artificial

<220>
<223>  VH CDR3

<400>  32

Tyr Thr Leu Tyr Tyr Gly Asn Pro Tyr Tyr Tyr Ala Met Asp Tyr
1               5                   10                  15

<210>  33
<211>  15
<212>  PRT
<213>  Artificial

<220>
<223>  VH CDR3

<400>  33

Phe Thr Leu Tyr His Gly Asn Pro Tyr Tyr Tyr Ala Met Asp Tyr
1               5                   10                  15

<210>  34
<211>  15
<212>  PRT
<213>  Artificial

<220>
<223>  VH CDR3

<400>  34

Tyr Thr Leu Tyr Tyr Gly Asn Pro Tyr Tyr Tyr Ala Met Glu Tyr
1               5                   10                  15

<210>  35
<211>  8
<212>  PRT
<213>  Artificial

<220>
<223>  VH CDR3

<400>  35

Arg Asn Phe Tyr Ala Met Asp Tyr
1               5

<210>  36
<211>  9
<212>  PRT
<213>  Artificial

<220>
<223>  VH CDR3

<400>  36

Gly Gly Arg Pro Leu Tyr Phe Asp Tyr
1               5


<210>  37
<211>  15
<212>  PRT
<213>  Artificial

<220>
<223>  VH CDR3


<220>
<221>  misc_feature
<222>  (1)..(1)
<223>  Xaa can be any naturally occurring amino acid

<220>
<221>  misc_feature
<222>  (5)..(7)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (11)..(11)
<223>  Xaa can be any naturally occurring amino acid

<220>
<221>  misc_feature
<222>  (14)..(14)
<223>  Xaa can be any naturally occurring amino acid

<400>  37

Xaa Thr Leu Tyr Xaa Xaa Xaa Pro Xaa Tyr Xaa Ala Met Xaa Tyr
1               5                   10                  15


<210>  38
<211>  15
<212>  PRT
<213>  Artificial

<220>
<223>  VH CDR3


<220>
<221>  misc_feature
<222>  (1)..(1)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (14)..(14)
<223>  Xaa can be any naturally occurring amino acid

<400>  38

Xaa Thr Leu Tyr Xaa Gly Asn Pro Tyr Tyr Tyr Ala Met Xaa Tyr
1               5                   10                  15


<210>  39
<211>  15
<212>  PRT
<213>  Artificial

<220>
<223>  VH CDR3


<220>
<221>  misc_feature
<222>  (1)..(1)
<223>  Xaa can be any naturally occurring amino acid

<220>
<221>  misc_feature
<222>  (5)..(6)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (14)..(14)
<223>  Xaa can be any naturally occurring amino acid

<400>  39

Xaa Thr Leu Tyr Xaa Xaa Asn Pro Xaa Tyr Tyr Ala Met Xaa Tyr
1               5                   10                  15


<210>  40
<211>  15
<212>  PRT
<213>  Artificial

<220>
<223>  VH CDR3


<220>
<221>  misc_feature
<222>  (1)..(1)
<223>  Xaa can be any naturally occurring amino acid

<220>
<221>  misc_feature

<222>  (5)..(5)
<223>  Xaa can be any naturally occurring amino acid

<220>
<221>  misc_feature
<222>  (7)..(7)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (11)..(11)
<223>  Xaa can be any naturally occurring amino acid

<220>
<221>  misc_feature
<222>  (14)..(14)
<223>  Xaa can be any naturally occurring amino acid

<400>  40

Xaa Thr Leu Tyr Xaa Gly Xaa Pro Xaa Tyr Xaa Ala Met Xaa Tyr
1               5                   10                  15


<210>  41
<211>  14
<212>  PRT
<213>  Artificial

<220>
<223>  VL CDR1

<400>  41

Arg Ser Ser Thr Gly Ala Val Thr Thr Ser Asn Tyr Ala Asn
1               5                   10


<210>  42
<211>  11
<212>  PRT
<213>  Artificial

<220>
<223>  VL CDR1

<400>  42

Arg Ala Ser Gln Asp Ile Ser Asn Tyr Leu Asn
1               5                   10


<210>  43
<211>  11
<212>  PRT
<213>  Artificial

<220>
<223>  VL CDR1

<400>  43

Ser Ala Ser Gln Gly Ile Ser Asn Tyr Leu Asn

1                 5                    10


<210>  44
<211>  10
<212>  PRT
<213>  Artificial

<220>
<223>  VL CDR1

<400>  44

Ser Ala Ser Ser Ser Val Ser Tyr Met His
1               5                   10


<210>  45
<211>  14
<212>  PRT
<213>  Artificial

<220>
<223>  VL CDR1


<220>
<221>  misc_feature
<222>  (1)..(2)
<223>  Xaa can be any naturally occurring amino acid

<220>
<221>  misc_feature
<222>  (4)..(5)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (13)..(14)
<223>  Xaa can be any naturally occurring amino acid

<400>  45

Xaa Xaa Ser Xaa Xaa Ala Val Thr Xaa Ser Asn Tyr Xaa Xaa
1               5                   10


<210>  46
<211>  12
<212>  PRT
<213>  Artificial

<220>
<223>  VL CDR1


<220>
<221>  misc_feature
<222>  (1)..(1)
<223>  Xaa can be any naturally occurring amino acid

<220>
<221>  misc_feature

<222>  (4)..(5)
<223>  Xaa can be any naturally occurring amino acid

<220>
<221>  misc_feature
<222>  (11)..(12)
<223>  Xaa can be any naturally occurring amino acid

<400>  46

Xaa Ala Ser Xaa Xaa Val Ile Ser Asn Tyr Xaa Xaa
1               5                   10


<210>  47
<211>  11
<212>  PRT
<213>  Artificial

<220>
<223>  VL CDR1


<220>
<221>  misc_feature
<222>  (1)..(1)
<223>  Xaa can be any naturally occurring amino acid

<220>
<221>  misc_feature
<222>  (5)..(5)
<223>  Xaa can be any naturally occurring amino acid

<400>  47

Xaa Ala Ser Gln Xaa Ile Ser Asn Tyr Leu Asn
1               5                   10


<210>  48
<211>  14
<212>  PRT
<213>  Artificial

<220>
<223>  VL CDR1


<220>
<221>  misc_feature
<222>  (1)..(2)
<223>  Xaa can be any naturally occurring amino acid

<220>
<221>  misc_feature
<222>  (4)..(5)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (13)..(13)
<223>  Xaa can be any naturally occurring amino acid

<400>  48

Xaa Xaa Ser Xaa Xaa Ala Val Thr Xaa Ser Asn Tyr Xaa Asn
1               5                   10


<210>  49
<211>  14
<212>  PRT
<213>  Artificial

<220>
<223>  VL CDR1


<220>
<221>  misc_feature
<222>  (1)..(2)
<223>  Xaa can be any naturally occurring amino acid

<220>
<221>  misc_feature
<222>  (4)..(5)
<223>  Xaa can be any naturally occurring amino acid

<220>
<221>  misc_feature
<222>  (13)..(14)
<223>  Xaa can be any naturally occurring amino acid

<400>  49

Xaa Xaa Ser Xaa Xaa Ala Val Thr Thr Ser Asn Tyr Xaa Xaa
1               5                   10


<210>  50
<211>  7
<212>  PRT
<213>  Artificial

<220>
<223>  VL CDR2

<400>  50

Gly Thr Asn Asn Arg Pro Pro
1               5


<210>  51
<211>  7
<212>  PRT
<213>  Artificial

<220>
<223>  VL CDR2

<400>  51

Gly Thr Asn Asn Arg Ala Pro
1               5


<210>  52
<211>  7
<212>  PRT
<213>  Artificial

<220>
<223>  VL CDR2

<400>  52

Tyr Thr Ser Arg Leu His Ser
1               5


<210>  53
<211>  7
<212>  PRT
<213>  Artificial

<220>
<223>  VL CDR2

<400>  53

Asp Thr Ser Lys Leu Ala Ser
1               5


<210>  54
<211>  7
<212>  PRT
<213>  Artificial

<220>
<223>  VL CDR2


<220>
<221>  misc_feature
<222>  (1)..(1)
<223>  Xaa can be any naturally occurring amino acid

<220>
<221>  misc_feature
<222>  (3)..(7)
<223>  Xaa can be any naturally occurring amino acid

<400>  54

Xaa Thr Xaa Xaa Xaa Xaa Xaa
1               5


<210>  55
<211>  7
<212>  PRT
<213>  Artificial

<220>
<223>  VL CDR2


<220>
<221>  misc_feature
<222>  (6)..(6)
<223>  Xaa can be any naturally occurring amino acid

<400>  55

Gly Thr Asn Asn Arg Xaa Pro
1               5

<210>  56
<211>  7
<212>  PRT
<213>  Artificial

<220>
<223>  VL CDR2


<220>
<221>  misc_feature
<222>  (1)..(1)
<223>  Xaa can be any naturally occurring amino acid

<220>
<221>  misc_feature
<222>  (4)..(4)
<223>  Xaa can be any naturally occurring amino acid

<220>
<221>  misc_feature
<222>  (6)..(6)
<223>  Xaa can be any naturally occurring amino acid

<400>  56

Xaa Thr Ser Xaa Leu Xaa Ser
1               5


<210>  57
<211>  7
<212>  PRT
<213>  Artificial

<220>
<223>  VL CDR2


<220>
<221>  misc_feature
<222>  (1)..(1)
<223>  Xaa can be any naturally occurring amino acid

<220>
<221>  misc_feature
<222>  (3)..(7)
<223>  Xaa can be any naturally occurring amino acid

<400>  57

Xaa Thr Xaa Xaa Xaa Xaa Xaa
1               5


<210>  58
<211>  7
<212>  PRT
<213>  Artificial

<220>
<223>  VL CDR2


<220>
<221>  misc_feature
<222>  (1)..(1)

<223>  Xaa can be any naturally occurring amino acid

<220>
<221>  misc_feature
<222>  (3)..(7)
<223>  Xaa can be any naturally occurring amino acid

<400>  58

Xaa Thr Xaa Xaa Xaa Xaa Xaa
1               5


<210>  59
<211>  9
<212>  PRT
<213>  Artificial

<220>
<223>  VL CDR3

<400>  59

Ala Leu Trp Tyr Ser Asn His Trp Val
1               5


<210>  60
<211>  9
<212>  PRT
<213>  Artificial

<220>
<223>  VL CDR3

<400>  60

Ala Leu Trp Phe Ser Asn His Trp Val
1               5


<210>  61
<211>  9
<212>  PRT
<213>  Artificial

<220>
<223>  VL CDR3

<400>  61

Gln Gln Gly Asn Thr Leu Pro Pro Thr
1               5


<210>  62
<211>  9
<212>  PRT
<213>  Artificial

<220>
<223>  VL CDR3

<400>  62

Gln Gln Tyr Ser Lys Leu Pro Arg Thr
1               5

<210>  63
<211>  9
<212>  PRT
<213>  Artificial

<220>
<223>  VL CDR3

<400>  63

Gln Gln Trp Ser Ser Asn Pro Pro Thr
1               5


<210>  64
<211>  9
<212>  PRT
<213>  Artificial

<220>
<223>  VL CDR3

<400>  64

Gln Gln Trp Ser Ser Asn Pro Leu Thr
1               5


<210>  65
<211>  9
<212>  PRT
<213>  Artificial

<220>
<223>  VL CDR3


<220>
<221>  misc_feature
<222>  (1)..(9)
<223>  Xaa can be any naturally occurring amino acid

<400>  65

Xaa Xaa Xaa Xaa Xaa Xaa Xaa Xaa Xaa
1               5


<210>  66
<211>  9
<212>  PRT
<213>  Artificial

<220>
<223>  VL CDR3


<220>
<221>  misc_feature
<222>  (4)..(4)
<223>  Xaa can be any naturally occurring amino acid

<400>  66

Ala Leu Trp Xaa Ser Asn His Trp Val
1               5

<210>  67
<211>  9
<212>  PRT
<213>  Artificial

<220>
<223>  VL CDR3


<220>
<221>  misc_feature
<222>  (3)..(6)
<223>  Xaa can be any naturally occurring amino acid

<220>
<221>  misc_feature
<222>  (8)..(8)
<223>  Xaa can be any naturally occurring amino acid

<400>  67

Gln Gln Xaa Xaa Xaa Xaa Pro Xaa Thr
1               5


<210>  68
<211>  9
<212>  PRT
<213>  Artificial

<220>
<223>  VL CDR3


<220>
<221>  misc_feature
<222>  (3)..(5)
<223>  Xaa can be any naturally occurring amino acid

<220>
<221>  misc_feature
<222>  (8)..(8)
<223>  Xaa can be any naturally occurring amino acid

<400>  68

Gln Gln Xaa Xaa Xaa Leu Pro Xaa Thr
1               5


<210>  69
<211>  9
<212>  PRT
<213>  Artificial

<220>
<223>  VL CDR3


<220>
<221>  misc_feature
<222>  (8)..(8)
<223>  Xaa can be any naturally occurring amino acid

<400>  69

Gln Gln Trp Ser Ser Asn Pro Xaa Thr

1               5


<210>  70
<211>  9
<212>  PRT
<213>  Artificial

<220>
<223>  VL CDR3


<220>
<221>  misc_feature
<222>  (1)..(9)
<223>  Xaa can be any naturally occurring amino acid

<400>  70

Xaa Xaa Xaa Xaa Xaa Xaa Xaa Xaa Xaa
1               5


<210>  71
<211>  9
<212>  PRT
<213>  Artificial

<220>
<223>  VL CDR3


<220>
<221>  misc_feature
<222>  (1)..(2)
<223>  Xaa can be any naturally occurring amino acid

<220>
<221>  misc_feature
<222>  (4)..(4)
<223>  Xaa can be any naturally occurring amino acid

<220>
<221>  misc_feature
<222>  (7)..(9)
<223>  Xaa can be any naturally occurring amino acid

<400>  71

Xaa Xaa Trp Xaa Ser Asn Xaa Xaa Xaa
1               5


<210>  72
<211>  15
<212>  DNA
<213>  Artificial

<220>
<223>  VH CDR1

<400>  72
aattacttga tagag                                                        15


<210>  73
<211>  15
<212>  DNA

<213>  Artificial

<220>
<223>  VH CDR1

<400>  73
gacacctata tacac                                                    15


<210>  74
<211>  15
<212>  DNA
<213>  Artificial

<220>
<223>  VH CDR1

<400>  74
aactatgcca tgtct                                                    15


<210>  75
<211>  15
<212>  DNA
<213>  Artificial

<220>
<223>  VH CDR1

<400>  75
raywmykhba trbmb                                                    15


<210>  76
<211>  15
<212>  DNA
<213>  Artificial

<220>
<223>  VH CDR1

<400>  76
raywmctwka tasas                                                    15


<210>  77
<211>  15
<212>  DNA
<213>  Artificial

<220>
<223>  VH CDR1

<400>  77
aaytaykysa trkmk                                                    15


<210>  78
<211>  51
<212>  DNA
<213>  Artificial

<220>
<223>  VH CDR2

<400>  78
gtgattaatc ctggaagtgg tggtactaac tacaatgaga agttcaaggg c            51

<210>  79
<211>  51
<212>  DNA
<213>  Artificial

<220>
<223>  VH CDR2

<400>  79
gtgattaatc ctggaagtgg tggtactaac tacaatgaga agttcaagga c          51


<210>  80
<211>  51
<212>  DNA
<213>  Artificial

<220>
<223>  VH CDR2

<400>  80
aggattgatc ctgcgattgg taatactaaa tatgacccga agttccaggg c          51


<210>  81
<211>  48
<212>  DNA
<213>  Artificial

<220>
<223>  VH CDR2

<400>  81
tccattaata gtggtggtta cacctactat ccagacagtg tgaagggc          48


<210>  82
<211>  48
<212>  DNA
<213>  Artificial

<220>
<223>  VH CDR2

<400>  82
tccattacta gtggcggtat tacctactat ccagacagtg tgaagggc          48


<210>  83
<211>  51
<212>  DNA
<213>  Artificial

<220>
<223>  VH CDR2


<220>
<221>  misc_feature
<222>  (15)..(15)
<223>  n is a, c, g, or t

<400>  83
dbsattrmtm stgsnaktgg tddyacywam tayvmhsmsa rkktsmaggr c          51


<210>  84

<211>  51
<212>  DNA
<213>  Artificial

<220>
<223>  VH CDR2

<400>  84
gtgattaatc ctggaagtgg tggtactaac tacaatgaga agttcaaggr c          51


<210>  85
<211>  51
<212>  DNA
<213>  Artificial

<220>
<223>  VH CDR2

<400>  85
rkgattratc ctgsraktgg trrtactaam tayraysmga agttcmaggr c          51


<210>  86
<211>  48
<212>  DNA
<213>  Artificial

<220>
<223>  VH CDR2

<400>  86
tccattamta gtggyggtww yacctactat ccagacagtg tgaagggc          48


<210>  87
<211>  51
<212>  DNA
<213>  Artificial

<220>
<223>  VH CDR2

<400>  87
kysattamtm stgghagtgg tddyacywac taymmwgasa rkktsaaggr c          51


<210>  88
<211>  45
<212>  DNA
<213>  Artificial

<220>
<223>  VH CDR3

<400>  88
tataccctct actatggtaa cccttattac tatgctatgg actac          45


<210>  89
<211>  45
<212>  DNA
<213>  Artificial

<220>
<223>  VH CDR3

<400>  89

tttaccctct atcatggtaa cccttattac tatgctatgg actac                    45

<210>  90
<211>  45
<212>  DNA
<213>  Artificial

<220>
<223>  VH CDR3

<400>  90
tataccctct actatggtaa cccttattac tatgctatgg agtac                    45

<210>  91
<211>  24
<212>  DNA
<213>  Artificial

<220>
<223>  VH CDR3

<400>  91
aggaatttct atgctatgga ctac                                           24

<210>  92
<211>  27
<212>  DNA
<213>  Artificial

<220>
<223>  VH CDR3

<400>  92
ggcggacgac ccctttactt tgactac                                        27

<210>  93
<211>  27
<212>  DNA
<213>  Artificial

<220>
<223>  VH CDR3

<400>  93
ggcggacggc ccctttactt tgactac                                        27

<210>  94
<211>  45
<212>  DNA
<213>  Artificial

<220>
<223>  VH CDR3

<220>
<221>  misc_feature
<222>  (21)..(21)
<223>  n is a, c, g, or t

<400>  94
twtaccctct aybryrgdmr nccyywytay twtgctatgg astac                    45

<210>  95
<211>  45
<212>  DNA
<213>  Artificial

<220>
<223>  VH CDR3

<400>  95
twtaccctct ayyatggtaa cccttattac tatgctatgg astac                45


<210>  96
<211>  45
<212>  DNA
<213>  Artificial

<220>
<223>  VH CDR3

<400>  96
twtaccctct ayyatrgkaa yccttwytay tatgctatgg astac                45


<210>  97
<211>  27
<212>  DNA
<213>  Artificial

<220>
<223>  VH CDR3

<400>  97
ggcggacgrc ccctttactt tgactac                                    27


<210>  98
<211>  45
<212>  DNA
<213>  Artificial

<220>
<223>  VH CDR3

<400>  98
twtaccctct aybryggwmr vccyywttac twtgctatgg astac                45


<210>  99
<211>  42
<212>  DNA
<213>  Artificial

<220>
<223>  VL CDR1

<400>  99
cgctcaagta ctggggctgt tacaactagt aactatgcca ac                   42


<210>  100
<211>  33
<212>  DNA
<213>  Artificial

<220>
<223>  VL CDR1

<400>  100
agggcaagtc aggacattag caattattta aac                                    33


<210>  101
<211>  33
<212>  DNA
<213>  Artificial

<220>
<223>  VL CDR1

<400>  101
agtgcaagtc agggcattag caattattta aac                                    33


<210>  102
<211>  30
<212>  DNA
<213>  Artificial

<220>
<223>  VL CDR1

<400>  102
agtgccagct caagtgtaag ttacatgcac                                        30


<210>  103
<211>  42
<212>  DNA
<213>  Artificial

<220>
<223>  VL CDR1

<400>  103
mgbkcmagyh mdrrbgctgt wacaaytagy aaytaydyvm ac                          42


<210>  104
<211>  36
<212>  DNA
<213>  Artificial

<220>
<223>  VL CDR1

<400>  104
agkgcmagyy mrrrygtaat tagyaattay wtrmac                                 36


<210>  105
<211>  33
<212>  DNA
<213>  Artificial

<220>
<223>  VL CDR1

<400>  105
agkgcaagtc aggrcattag caattattta aac                                    33


<210>  106
<211>  42
<212>  DNA

<213>  Artificial

<220>
<223>  VL CDR1

<400>  106
mgbkcaagtm mkgrsgctgt tacaaytagy aaytatkyma ac                          42


<210>  107
<211>  42
<212>  DNA
<213>  Artificial

<220>
<223>  VL CDR1

<400>  107
mgykcmagyw cwrgkgctgt wacaactagt aactayrysm ac                          42


<210>  108
<211>  21
<212>  DNA
<213>  Artificial

<220>
<223>  VL CDR2

<400>  108
ggtaccaaca accgacctcc a                                                 21


<210>  109
<211>  21
<212>  DNA
<213>  Artificial

<220>
<223>  VL CDR2

<400>  109
ggtaccaaca accgagctcc a                                                 21


<210>  110
<211>  21
<212>  DNA
<213>  Artificial

<220>
<223>  VL CDR2

<400>  110
tacacatcaa gattacactc a                                                 21


<210>  111
<211>  21
<212>  DNA
<213>  Artificial

<220>
<223>  VL CDR2

<400>  111
tacacatcaa gtttacactc a                                                 21

<210>  112
<211>  21
<212>  DNA
<213>  Artificial

<220>
<223>  VL CDR2

<400>  112
gacacatcca aactggcttc t                                                21


<210>  113
<211>  21
<212>  DNA
<213>  Artificial

<220>
<223>  VL CDR2

<400>  113
kryacmwmma rhykrsmyyc w                                                21


<210>  114
<211>  21
<212>  DNA
<213>  Artificial

<220>
<223>  VL CDR2

<400>  114
ggtaccaaca accgasctcc a                                                21


<210>  115
<211>  21
<212>  DNA
<213>  Artificial

<220>
<223>  VL CDR2

<400>  115
kacacatcma rwytrsmytc w                                                21


<210>  116
<211>  21
<212>  DNA
<213>  Artificial

<220>
<223>  VL CDR2

<400>  116
tacacatcaa gwttacactc a                                                21


<210>  117
<211>  21
<212>  DNA
<213>  Artificial

<220>
<223>  VL CDR2

<400>  117
kryacmwmma rhykasmyyc a                                                21

<210>  118
<211>  21
<212>  DNA
<213>  Artificial

<220>
<223>  VL CDR2

<400>  118
gryacmwmca amckrsctyc w                                                21

<210>  119
<211>  27
<212>  DNA
<213>  Artificial

<220>
<223>  VL CDR3

<400>  119
gctctatggt acagcaacca ttgggtg                                          27

<210>  120
<211>  27
<212>  DNA
<213>  Artificial

<220>
<223>  VL CDR3

<400>  120
gctctatggt acagcaacca ctgggtg                                          27

<210>  121
<211>  27
<212>  DNA
<213>  Artificial

<220>
<223>  VL CDR3

<400>  121
gctctatggt tcagcaacca ttgggtg                                          27

<210>  122
<211>  27
<212>  DNA
<213>  Artificial

<220>
<223>  VL CDR3

<400>  122
caacagggta atacgcttcc gtggacg                                          27

<210>  123
<211>  27
<212>  DNA

<213>  Artificial

<220>
<223>  VL CDR3

<400>  123
cagcagtata gtaagcttcc tcggacg                                          27


<210>  124
<211>  27
<212>  DNA
<213>  Artificial

<220>
<223>  VL CDR3

<400>  124
cagcagtgga gtagtaaccc accaacg                                          27


<210>  125
<211>  27
<212>  DNA
<213>  Artificial

<220>
<223>  VL CDR3

<400>  125
cagcagtgga gtagtaaccc gctcacg                                          27


<210>  126
<211>  27
<212>  DNA
<213>  Artificial

<220>
<223>  VL CDR3


<220>
<221>  misc_feature
<222>  (21)..(21)
<223>  n is a, c, g, or t

<400>  126
smdcwrkrkw dyavbmwycm nybvryg                                          27


<210>  127
<211>  27
<212>  DNA
<213>  Artificial

<220>
<223>  VL CDR3

<400>  127
gctctatggt wcagcaacca ytgggtg                                          27


<210>  128
<211>  27
<212>  DNA
<213>  Artificial

<220>
<223>  VL CDR3

<400>  128
carcagkrka rtavkmwycc dybvacg                                          27


<210>  129
<211>  27
<212>  DNA
<213>  Artificial

<220>
<223>  VL CDR3

<400>  129
carcagkrta rtamgcttcc kyggacg                                          27


<210>  130
<211>  27
<212>  DNA
<213>  Artificial

<220>
<223>  VL CDR3

<400>  130
cagcagtgga gtagtaaccc rcymacg                                          27


<210>  131
<211>  27
<212>  DNA
<213>  Artificial

<220>
<223>  VL CDR3

<400>  131
smdcwrkrkw dyavsmwycm byggryg                                          27


<210>  132
<211>  27
<212>  DNA
<213>  Artificial

<220>
<223>  VL CDR3


<220>
<221>  misc_feature
<222>  (21)..(21)
<223>  n is a, c, g, or t

<400>  132
smkcwrtggw dyagyaaccm nybvryg                                          27


<210>  133
<211>  12
<212>  PRT
<213>  Artificial

<220>
<223>  yol linker

<400>  133

Lys Leu Glu Glu Gly Glu Phe Ser Glu Ala Arg Val
1               5                   10


<210>  134
<211>  12
<212>  PRT
<213>  Artificial

<220>
<223>  my-linker

<400>  134

Ile Leu Glu Glu Gly Glu Phe Ser Glu Ala Gly Cys
1               5                   10


<210>  135
<211>  12
<212>  PRT
<213>  Artificial

<220>
<223>  Linker (consensus)


<220>
<221>  misc_feature
<222>  (1)..(1)
<223>  Xaa can be any naturally occurring amino acid

<220>
<221>  misc_feature
<222>  (11)..(12)
<223>  Xaa can be any naturally occurring amino acid

<400>  135

Xaa Leu Glu Glu Gly Glu Phe Ser Glu Ala Xaa Xaa
1               5                   10


<210>  136
<211>  5
<212>  PRT
<213>  Artificial

<220>
<223>  GS-linker (part)

<400>  136

Gly Gly Gly Gly Ser
1               5


<210>  137
<211>  35
<212>  DNA
<213>  Artificial

<220>
<223>  linker

<400>  137
aagcttgaag aaggtgaatt ttcagaagca cgcgt                                35


<210>  138
<211>  16
<212>  PRT
<213>  Artificial

<220>
<223>  VH CDR2


<220>
<221>  misc_feature
<222>  (1)..(1)
<223>  Xaa can be any naturally occurring amino acid

<220>
<221>  misc_feature
<222>  (3)..(5)
<223>  Xaa can be any naturally occurring amino acid

<220>
<221>  misc_feature
<222>  (7)..(7)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (11)..(16)
<223>  Xaa can be any naturally occurring amino acid

<400>  138

Xaa Ile Xaa Xaa Xaa Gly Xaa Thr Xaa Tyr Xaa Xaa Xaa Xaa Xaa Xaa
1               5                   10                  15


<210>  139
<211>  16
<212>  PRT
<213>  Artificial

<220>
<223>  VH CDR2


<220>
<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<220>
<221>  misc_feature
<222>  (7)..(7)
<223>  Xaa can be any naturally occurring amino acid

<220>
<221>  misc_feature
<222>  (9)..(9)
<223>  Xaa can be any naturally occurring amino acid

<220>
<221>  misc_feature
<222>  (11)..(14)
<223>  Xaa can be any naturally occurring amino acid

<220>
<221>  misc_feature
<222>  (16)..(16)
<223>  Xaa can be any naturally occurring amino acid

<400>  139

Xaa Ile Xaa Xaa Gly Gly Xaa Thr Xaa Tyr Xaa Xaa Xaa Xaa Lys Xaa
1               5                   10                  15


<210>  140
<211>  8
<212>  PRT
<213>  Artificial

<220>
<223>  VH CDR3


<220>
<221>  misc_feature
<222>  (1)..(3)
<223>  Xaa can be any naturally occurring amino acid

<220>
<221>  misc_feature
<222>  (7)..(7)
<223>  Xaa can be any naturally occurring amino acid

<400>  140

Xaa Xaa Xaa Tyr Ala Met Xaa Tyr
1               5


<210>  141
<211>  9
<212>  PRT
<213>  Artificial

<220>
<223>  VH CDR3


<220>
<221>  misc_feature
<222>  (1)..(3)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (7)..(8)

<223>  Xaa can be any naturally occurring amino acid

<400>  141

Xaa Xaa Xaa Pro Xaa Tyr Xaa Xaa Tyr
1               5


<210>  142
<211>  8
<212>  PRT
<213>  Artificial

<220>
<223>  VH CDR3


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (7)..(7)
<223>  Xaa can be any naturally occurring amino acid

<400>  142

Xaa Asn Xaa Tyr Ala Met Xaa Tyr
1               5


<210>  143
<211>  9
<212>  PRT
<213>  Artificial

<220>
<223>  VH CDR3


<220>
<221>  misc_feature
<222>  (1)..(1)
<223>  Xaa can be any naturally occurring amino acid

<220>
<221>  misc_feature
<222>  (3)..(3)
<223>  Xaa can be any naturally occurring amino acid

<220>
<221>  misc_feature
<222>  (5)..(5)
<223>  Xaa can be any naturally occurring amino acid

<220>
<221>  misc_feature
<222>  (7)..(8)
<223>  Xaa can be any naturally occurring amino acid

<400>  143

Xaa Gly Xaa Pro Xaa Tyr Xaa Xaa Tyr
1               5

<210>  144
<211>  11
<212>  PRT
<213>  Artificial

<220>
<223>  VL CDR1

<220>
<221>  misc_feature
<222>  (1)..(2)
<223>  Xaa can be any naturally occurring amino acid

<220>
<221>  misc_feature
<222>  (4)..(6)
<223>  Xaa can be any naturally occurring amino acid

<220>
<221>  misc_feature
<222>  (10)..(11)
<223>  Xaa can be any naturally occurring amino acid

<400>  144

Xaa Xaa Ser Xaa Xaa Xaa Ser Asn Tyr Xaa Xaa
1               5                   10

<210>  145
<211>  10
<212>  PRT
<213>  Artificial

<220>
<223>  VL CDR1

<220>
<221>  misc_feature
<222>  (1)..(2)
<223>  Xaa can be any naturally occurring amino acid

<220>
<221>  misc_feature
<222>  (4)..(5)
<223>  Xaa can be any naturally occurring amino acid

<220>
<221>  misc_feature
<222>  (9)..(10)
<223>  Xaa can be any naturally occurring amino acid

<400>  145

Xaa Xaa Ser Xaa Xaa Val Ser Tyr Xaa Xaa
1               5                   10

<210>  146
<211>  10
<212>  PRT

<213>  Artificial

<220>
<223>  VL CDR1


<220>
<221>  misc_feature
<222>  (1)..(1)
<223>  Xaa can be any naturally occurring amino acid

<220>
<221>  misc_feature
<222>  (4)..(5)
<223>  Xaa can be any naturally occurring amino acid

<220>
<221>  misc_feature
<222>  (9)..(10)
<223>  Xaa can be any naturally occurring amino acid

<400>  146

Xaa Ala Ser Xaa Xaa Val Ser Tyr Xaa Xaa
1               5                   10


<210>  147
<211>  11
<212>  PRT
<213>  Artificial

<220>
<223>  VL CDR1


<220>
<221>  misc_feature
<222>  (1)..(2)
<223>  Xaa can be any naturally occurring amino acid

<220>
<221>  misc_feature
<222>  (4)..(6)
<223>  Xaa can be any naturally occurring amino acid

<220>
<221>  misc_feature
<222>  (10)..(10)
<223>  Xaa can be any naturally occurring amino acid

<400>  147

Xaa Xaa Ser Xaa Xaa Xaa Ser Asn Tyr Xaa Asn
1               5                   10


<210>  148
<211>  11
<212>  PRT
<213>  Artificial

<220>
<223>  VL CDR1


<220>
<221>  misc_feature

<222>  (1)..(2)
<223>  Xaa can be any naturally occurring amino acid

<220>
<221>  misc_feature
<222>  (4)..(5)
<223>  Xaa can be any naturally occurring amino acid

<220>
<221>  misc_feature
<222>  (10)..(11)
<223>  Xaa can be any naturally occurring amino acid

<400>  148

Xaa Xaa Ser Xaa Xaa Val Ser Asn Tyr Xaa Xaa
1               5                   10